# FASCICULE DE BREVET EUROPEEN

(11) **EP 1 566 453 B1**
(45) Date de publication et mention de la délivrance du brevet: **09.09.2015**
(21) Numéro de dépôt: 05003839.7
(22) Date de dépôt: 11.03.1999
(51) Int. Cl.: C12Q 1/68

(54) **Criblage différentiel qualitatif**
Qualitatives differentielles Spleissen
Qualitative differential splicing

(30) Priorité: 11.03.1998 FR 9802997
(43) Date de publication de la demande: 24.08.2005
(62) Demande divisionnaire de: 99909002.0
(73) Titulaire: Diaxonhit, 75013 Paris (FR)
(72) Inventeur: Schweighoffer, Fabien, 94300 Vincennes (FR); Bracco, Laurent, 75013 Paris (FR); Tocque, Bruno, 92400 Courbevoie (FR)
(74) Mandataire: Becker, Philippe

(56) Documents cités:
- WO-A-95/27052
- WO-A-96/26272
- WO-A-98/02576
- FR-A- 2 664 287
- COOPER D L ET AL: "GENE THERAPY ADVANCES: UTILIZATION OF ALTERNATIVE SPLICING AS A CONTROL ELEMENT IN THE CHIMERIC ENZYME/PRODRUG THERAPY (CEPT) APPROACH TO PRIMARY AND METASTATIC TUMORS" JOURNAL OF CLINICAL LIGAND ASSAY, CLINICAL LIGAND ASSAY SOCIETY, WAYNE, MI, US, vol. 19, no. 1, 1 janvier 1996 (1996-01-01), pages 80-84, XP002038774 ISSN: 1081-1672
- ALPHEY L: "PCR-based method isolation of full-length clones and splice variants from cDNA libraries" BIOTECHNIQUES, INFORMA LIFE SCIENCES PUBLISHING, WESTBOROUGH, MA, US, vol. 22, 1 janvier 1997 (1997-01-01), page 481/482,484,486, XP002087172 ISSN: 0736-6205
- SCHENA M ET AL: "QUANTITATIVE MONITORING OF GENE EXPRESSION PATTERNS WITH A COMPLEMENTARY DNAMICROARRAY" SCIENCE, AMERICAN ASSOCIATION FOR THE ADVANCEMENT OF SCIENCE, US, WASHINGTON, DC, vol. 270, no. 5235, 20 octobre 1995 (1995-10-20), pages 467-470, XP000644675 ISSN: 0036-8075
- PERRET E ET AL: "Improved differential screening approach to analyse transcriptional variations in organized cDNA libraries" GENE, ELSEVIER, AMSTERDAM, NL, vol. 208, no. 2, 22 février 1998 (1998-02-22), pages 103-115, XP004114934 ISSN: 0378-1119
- ARDLEY H C ET AL: "Rapid isolation of genomic clones for individual members of human multigene families: Identification and localisation of UBE2L4, a novel member of a ubiquitin conjugating enzyme dispersed gene family" CYTOGENETICS AND CELL GENETICS, BASEL, CH, vol. 79, 1 janvier 1997 (1997-01-01), pages 188-192, XP002087173 ISSN: 0301-0171
- ANONYMOUS: "Capitalizing on the human spliceosome" NATURE BIOTECHNOLOGY, vol. 19, no. Supplement, 2001, pages BE12-BE13, XP002548225
- CRONIN M T ET AL: "Cystic fibrosis mutation detection by hybridization to light-generated DNA probe arrays", HUMAN MUTATION, JOHN WILEY & SONS, INC, US, vol. 7, no. 3, 1 January 1996 (1996-01-01) , pages 244-255, XP008030268, ISSN: 1059-7794, DOI: 10.1002/(SICI)1098-1004(1996)7:3<244::AID- HUMU9>3.0.CO;2-A
- DELANEY S J ET AL: "Alternative Splicing of the First Nucleotide Binding Fold of CFTR in Mouse Testes Is Associated with Specific Stages of Spermatogenesis", GENOMICS, ACADEMIC PRESS, SAN DIEGO, US, vol. 20, no. 3, 1 April 1994 (1994-04-01), pages 517-518, XP024796960, ISSN: 0888-7543, DOI: 10.1006/GENO.1994.1214 [retrieved on 1994-04-01]
- SHIUE L: "IDENTIFICATION OF CANDIDATE GENES FOR DRUG DISCOVERY BY DIFFERENTIAL DISPLAY", DRUG DEVELOPMENT RESEARCH, NEW YORK, NY, US, vol. 41, 1 January 1997 (1997-01-01), pages 142-159, XP000893087, ISSN: 0272-4391, DOI: 10.1002/(SICI)1098-2299(199707/08)41:3/4<1 42::AID-DDR5>3.0.CO;2-L
- JOHNSON JASON M ET AL: "Genome-wide survey of human alternative pre-mRNA splicing with exon junction microarrays", SCIENCE, AMERICAN ASSOCIATION FOR THE ADVANCEMENT OF SCIENCE, US, vol. 302, no. 5653, 19 December 2003 (2003-12-19), pages 2141-2144, XP002458857, ISSN: 0036-8075, DOI: 10.1126/SCIENCE.1090100

### LISTE DE SEQUENCES

<110> EXONHIT THERAPEUTICS SA
<120> CRIBLAGE DIFFERENTIEL QUALITATIF
<130> B3898B - PB/KM
<140>
   <141>
<150> 9802997
   <151> 1998-03-11
<160> 16
<170> PatentIn Ver. 2.1
<210> 1
   <211> 23
   <212> ADN
   <213> Séquence artificielle
<220>
   <223> Description de la séquence artificielle: OLIGO
<400> 1
   gagaagcgtt atnnnnnnna ggn 23
<210> 2
   <211> 24
   <212> ADN
   <213> Séquence artificielle
<220>
   <223> Description de la séquence artificielle: OLIGO
<400> 2
   gagaagcgtt atnnnnnnnn tccc 24
<210> 3
   <211> 23
   <212> ADN
   <213> Séquence artificielle
<220>
   <223> Description de la séquence artificielle: OLIGO
<400> 3
   gagaagcgtt atnnnnnnnn nnn 23
<210> 4
   <211> 20
   <212> ADN
   <213> Séquence artificielle
<220>
   <223> Description de la séquence artificielle: OLIGO
<400> 4
   gagaagcgtt atnnnnncca 20
<210> 5
   <211> 66
   <212> ADN
   <213> Homo sapiens
<400> 5
   ccacacctgg ccagtatgtg ctcactggct tgcagagtgg gcagccagcc taagcatttg 60
   cactgg 66
<210> 6
   <211> 23
   <212> ADN
   <213> Séquence artificielle
<220>
   <223> Description de la séquence artificielle: OLIGO
<400> 6
   gggacctgtt tgacatgaag ccc 23
<210> 7
   <211> 22
   <212> ADN
   <213> Séquence artificielle
<220>
   <223> Description de la séquence artificielle: OLIGO
<400> 7
   cagtttccgc tccacaggtt gc 22
<210> 8
   <211> 96
   <212> ADN
   <213> Homo sapiens
<400> 8
   gtacgggaga gcacgaccac acctggccag tatgtgctca ctggcttgca gagtgggcag 60
   cctaagcatt tgctactggt ggaccctgag ggtgtg                            96
<210> 9
   <211> 441
   <212> PRT
   <213> Homo sapiens
<400> 9
   Met Asn Lys Leu Ser Gly Gly Gly Gly Arg Arg Thr Arg Val Glu Gly
   1               5                   10                  15
   Gly Gln Leu Gly Gly Glu Glu Trp Thr Arg His Gly Ser Phe Val Asn
               20                  25                  30
   Lys Pro Thr Arg Gly Trp Leu His Pro Asn Asp Lys Val Met Gly Pro
           35                  40                  45
   Gly Val Ser Tyr Leu Val Arg Tyr Met Gly Cys Val Glu Val Leu Gln
       50                  55                  60
   Ser Met Arg Ala Leu Asp Phe Asn Thr Arg Thr Gln Val Thr Arg Glu
   65                  70                  75                  80
   Ala Ile Ser Leu Val Cys Glu Ala Val Pro Gly Ala Lys Gly Ala Thr
                   85                  90                  95
   Arg Arg Arg Lys Pro Cys Ser Arg Pro Leu Ser Ser Ile Leu Gly Arg
               100                 105                 110
   Ser Asn Leu Lys Phe Ala Gly Met Pro Ile Thr Leu Thr Val Ser Thr
           115                 120                 125
   Ser Ser Leu Asn Leu Met Ala Ala Asp Cys Lys Gln Ile Ile Ala Asn
       130                 135                 140
   His His Met Gln Ser Ile Ser Phe Ala Ser Gly Gly Asp Pro Asp Thr
   145                 150                 155                 160
   Ala Glu Tyr Val Ala Tyr Val Ala Lys Asp Pro Val Asn Gln Arg Ala
                   165                 170                 175
   Cys His Ile Leu Glu Cys Pro Glu Gly Leu Ala Gln Asp Val Ile Ser
               180                 185                 190
   Thr Ile Gly Gln Ala Phe Glu Leu Arg Phe Lys Gln Tyr Leu Arg Asn
           195                 200                 205
   Pro Pro Lys Leu Val Thr Pro His Asp Arg Met Ala Gly Phe Asp Gly
       210                 215                 220
   Ser Ala Trp Asp Glu Glu Glu Glu Glu Pro Pro Asp His Gln Tyr Tyr
   225                 230                 235                 240
   Asn Asp Phe Pro Gly Lys Glu Pro Pro Leu Gly Gly Val Val Asp Met
                   245                 250                 255
   Arg Leu Arg Glu Gly Ala Ala Pro Gly Ala Ala Arg Pro Thr Ala Pro
               260                 265                 270
   Asn Ala Gln Thr Pro Ser His Leu Gly Ala Thr Leu Pro Val Gly Gln
           275                 280                 285
   Pro Val Gly Gly Asp Pro Glu Val Arg Lys Gln Met Pro Pro Pro Pro
       290                 295                 300
   Pro Cys Pro Gly Arg Glu Leu Phe Asp Asp Pro Ser Tyr Val Asn Val
   305                 310                 315                 320
   Gln Asn Leu Asp Lys Ala Arg Gln Ala Val Gly Gly Ala Gly Pro Pro
                   325                 330                 335
   Asn Pro Ala Ile Asn Gly Ser Ala Pro Arg Asp Leu Phe Asp Met Lys
               340                 345                 350
   Pro Phe Glu Asp Ala Leu Arg Val Pro Pro Pro Pro Gln Ser Val Ser
           355                 360                 365
   Met Ala Glu Gln Leu Arg Gly Glu Pro Trp Phe His Gly Lys Leu Ser
       370                 375                 380
   Arg Arg Glu Ala Glu Ala Leu Leu Gln Leu Asn Gly Asp Phe Leu Val
   385                 390                 395                 400
   Arg Thr Lys Asp His Arg Phe Glu Ser Val Ser His Leu Ile Ser Tyr
                   405                 410                 415
   His Met Asp Asn His Leu Pro Ile Ile Ser Ala Gly Ser Glu Leu Cys
               420                 425                 430
   Leu Gln Gln Pro Val Glu Arg Lys Leu
           435                 440
<210> 10
   <211> 1326
   <212> ADN
   <213> Homo sapiens
<400> 10
   atgaacaagc tgagtggagg cggcgggcgc aggactcggg tggaaggggg ccagcttggg 60
   ggcgaggagt ggacccgcca cgggagcttt gtcaataagc ccacgcgggg ctggctgcat 120
   cccaacgaca aagtcatggg acccggggtt tcctacttgg ttcggtacat gggttgtgtg 180
   gaggtcctcc agtcaatgcg tgccctggac ttcaacaccc ggactcaggt caccagggag 240
   gccatcagtc tggtgtgtga ggctgtgccg ggtgctaagg gggcgacaag gaggagaaag 300
   ccctgtagcc gcccgctcag ctctatcctg gggaggagta acctgaaatt tgctggaatg 360
   ccaatcactc tcaccgtctc caccagcagc ctcaacctca tggccgcaga ctgcaaacag 420
   atcatcgcca accaccacat gcaatctatc tcatttgcat ccggcgggga tccggacaca 480
   gccgagtatg tcgcctatgt tgccaaagac cctgtgaatc agagagcctg ccacattctg 540
   gagtgtcccg aagggcttgc ccaggatgtc atcagcacca ttggccaggc cttcgagttg 600
   cgcttcaaac aatacctcag gaacccaccc aaactggtca cccctcatga caggatggct 660
   ggctttgatg gctcagcatg ggatgaggag gaggaagagc cacctgacca tcagtactat 720
   aatgacttcc cggggaagga accccccttg gggggggtgg tagacatgag gcttcgggaa 780
   ggagccgctc caggggctgc tcgacccact gcacccaatg cccagacccc cagccacttg 840
   ggagctacat tgcctgtagg acagcctgtt gggggagatc cagaagtccg caaacagatg 900
   ccacctccac caccctgtcc aggcagagag ctttttgatg atccctccta tgtcaacgtc 960
   cagaacctag acaaggcccg gcaagcagtg ggtggtgctg ggccccccaa tcctgctatc 1020
   aatggcagtg caccccggga cctgtttgac atgaagccct tcgaagatgc tcttcgggtg 1080
   cctccacctc cccagtcggt gtccatggct gagcagctcc gaggggagcc ctggttccat 1140
   gggaagctga gccggcggga ggctgaggca ctgctgcagc tcaatgggga cttcttggtt 1200
   cggactaagg atcaccgctt tgaaagtgtc agtcacctta tcagctacca catggacaat 1260
   cacttgccca tcatctctgc gggcagcgaa ctgtgtctac agcaacctgt ggagcggaaa 1320
   ctgtga                                                            1326
<210> 11
   <211> 19
   <212> ADN
   <213> Séquence artificielle
   b
<220>
   <223> Description de la séquence artificielle: OLIGO
<400> 11
   tgcccaaatc aacaagagc 19
<210> 12
   <211> 19
   <212> ADN
   <213> Séquence artificielle
<220>
   <223> Description de la séquence artificielle: OLIGO
<400> 12
   cccctgacaa gcctgaata 19
<210> 13
   <211> 24
   <212> ADN
   <213> Séquence artificielle
<220>
   <223> Description de la séquence artificielle: OLIGO
<400> 13
   atgtctcaga gcaaccggga gctg 24
<210> 14
   <211> 24
   <212> ADN
   <213> Séquence artificielle
<220>
   <223> Description de la séquence artificielle: OLIGO
<400> 14
   gtggctccat tcaccgcggg gctg 24
<210> 15
   <211> 19
   <212> ADN
   <213> Séquence artificielle
<220>
   <223> Description de la séquence artificielle: OLIGO
<400> 15
   tgccaagaag ggaaggagt 19
<210> 16
   <211> 20
   <212> ADN
   <213> Séquence artificielle
<220>
   <223> Description de la séquence artificielle: OLIGO
<400> 16
   tgtcatgact ccagcaatag 20

## Description

Cette invention se rapporte aux domaines techniques de la biotechnologie, de la médecine, de la biologie et de la biochimie. Ses applications concernent les domaines de la santé humaine, animale et végétale. Plus particulièrement, l'invention permet d'identifier des séquences d'acides nucléiques permettant de concevoir de nouveaux cribles pour molécules d'intérêt thérapeutique, de nouveaux outils de thérapie génique ainsi que d'apporter des indications sur le potentiel toxique et le suivi d'efficacité de molécules et des informations de pharmacogénomique.

La présente invention décrit notamment une série de techniques originales d'identification de séquences d'acides nucléiques basée sur la mise en évidence des différences qualitatives entre les ARN issus de deux contextes différents que l'on désire comparer, en particulier issus d'un tissu ou d'un organe malade et leur équivalent sain. Plus précisément, ces techniques sont destinées à cloner spécifiquement les introns et les exons alternatifs épissés différentiellement entre une situation pathologique et un état sain ou entre deux situations physiologiques que l'on désire comparer. Ces différences qualitatives au sein des ARNs peuvent également provenir d'altération(s) du génome, de type insertions ou délétions dans des régions qui seront transcrites en ARN. Cette série de techniques est identifiée par l'acronyme DATAS : Differential Analysis of Transcripts with Alternative Splicing.

La caractérisation des altérations de l'expression génétique qui président ou sont associées à une pathologie donnée suscite un espoir important de découvrir de nouvelles cibles thérapeutiques et de nouveaux outils diagnostiques. Toutefois, l'identification d'une séquence d'ADN génomique ou complémentaire, qu'elle ait lieu par clonage positionnel ou par des techniques de criblage différentiel quantitatif, n'apporte que peu ou pas d'information sur la fonction et encore moins sur les domaines fonctionnels mis en jeu dans les dérégulations liées à la pathologie étudiée. La présente invention décrit une série de techniques originales qui visent à identifier les différences d'épissages des ARNs qui existent entre deux situations physiopathologiques distinctes. L'identification de ces différences apporte des informations sur les différences qualitatives et non sur les différences quantitatives comme c'est le cas pour les techniques décrites jusqu'à présent. L'ensemble des techniques présentées dans la présente invention sont donc regroupées sous l'appellation "criblage différentiel qualitatif" ou DATAS. Les méthodes de l'invention sont utilisables pour l'identification de nouvelles cibles ou produits thérapeutiques, pour la préparation d'outils de recherche génétique et/ou d'outils de diagnostic, pour la construction de banques d'acides nucléiques, et dans des méthodes de détermination du profil toxicologique ou de l'efficacité d'un composé par exemple.

A cet égard, l'invention est relative à des compositions ou banques d'acides nucléiques profilées, représentatives des différences qualitatives existant entre deux échantillons biologiques.

L'invention concerne plus particulièrement une composition telle que définie dans les revendications.

Plus généralement, l'invention décrit des compositions d'acides nucléiques comprenant essentiellement des acides nucléiques correspondant aux épissages alternatifs distinguant deux situations physiologiques. Plus particulièrement, ces acides nucléiques correspondent aux épissages alternatifs identifiés dans un échantillon biologique test et non présents dans le même échantillon biologique dans une situation de référence. L'invention décrit également l'utilisation de ces compositions d'acides nucléiques comme produit diagnostic, ou comme outil de criblage de molécules actives, comme indiqué ci-après.

### Génération de banques qualitatives

A cet égard, l'invention décrit un procédé de préparation d'une banque d'acides nucléiques représentatifs d'un état physiologique donné d'un échantillon biologique.

La présente invention peut être appliquée à tous types d'échantillons biologiques. En particulier, l'échantillon biologique peut être toute cellule, organe, tissu, prélèvement, biopsie, etc., contenant des acides nucléiques. S'agissant d'un organe, tissu ou biopsie, ils sont éventuellement mis en culture de manière à permettre l'accès aux cellules qui les composent. Il peut s'agir d'échantillons provenant de mammifères (en particulier l'homme), de végétaux, de bactéries ou de cellules eucaryotes inférieures (levures, cellules fongiques, etc.). Des exemples de matériels sont en particulier une biopsie de tumeur, une biopsie de plaques neurodégénératives ou d'aires cérébrales présentant des atteintes neurodégénératives, un échantillon de peau, un échantillon de cellules sanguines obtenues après prise de sang, une biopsie colorectale, des biopsies issues de lavages pulmonaires, etc. Des exemples de cellules sont notamment les cellules musculaires, hépatiques, fibroblastes, nerveuses, de l'épiderme, du derme, des cellules sanguines comme les lymphocytes B, T, les mastocytes, les monocytes, les granulocytes, les macrophages.

A titre illustratif, les situations physiologiques étudiées peuvent être les suivantes :

| SITUATION A | SITUATION B |
|---|---|
| échantillon sain | échantillon pathologique |
| échantillon sain | échantillon apoptotique |
| échantillon sain | échantillon après infection virale |
| échantillon sensible à X | échantillon résistant à X |
| échantillon non traité | échantillon traité (par exemple par composé toxique) |
| échantillon non différencié | échantillon ayant subi une différentiation cellulaire ou tissulaire |

Ce procédé comprend avantageusement le clonage d'acides nucléiques représentatifs des marqueurs qualitatifs d'expression génétiques (par exemple des épissages alternatifs) dudit état physiologique et non présents dans un état de référence, dans des banques spécifiques de différences qualitatives qui existent entre les 2 états étudiés.

Ces banques sont constituées d'ADNc insérés dans des vecteurs plasmidiques ou phagiques. Ces banques peuvent être présentées sur des filtres de nitrocellulose ou tout autre support connu de l'homme de l'art, tels des chips ou biopuces.

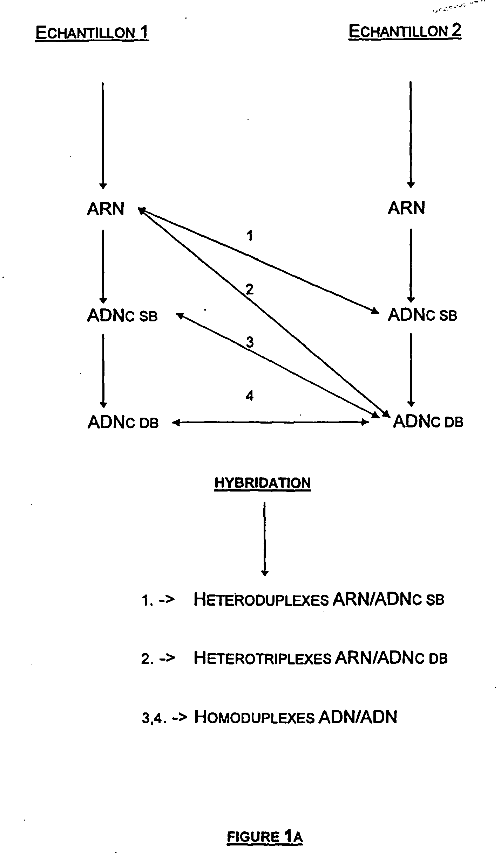

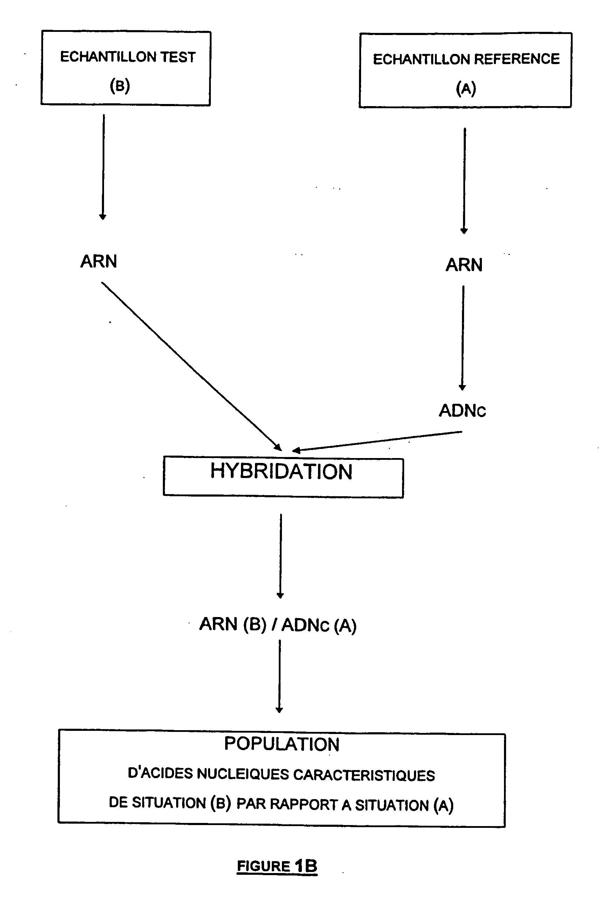

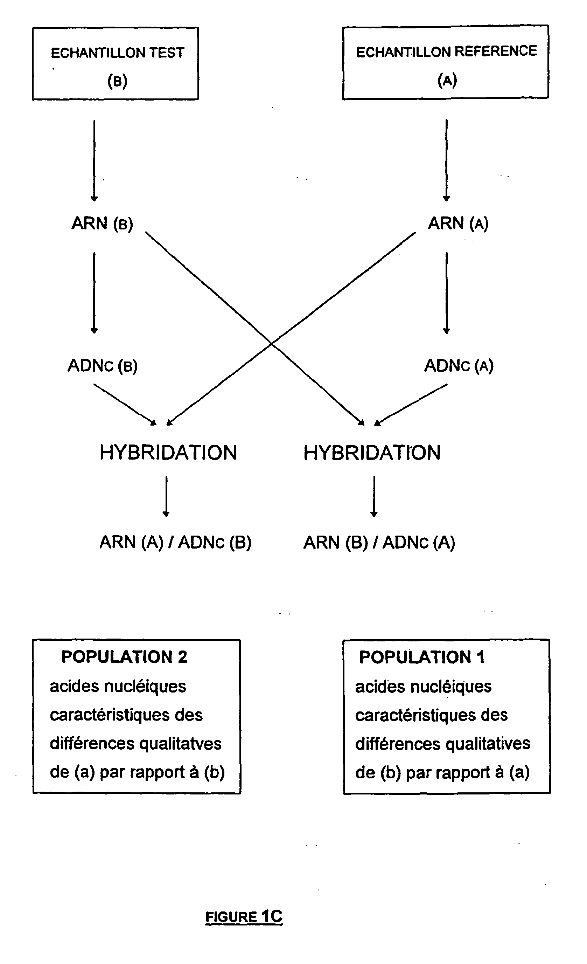

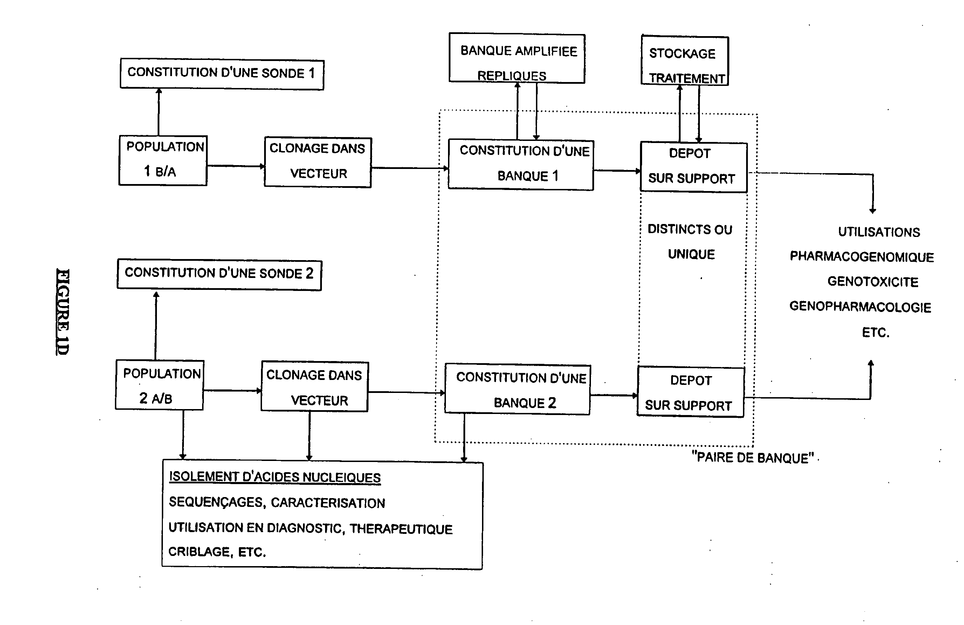

L'une des caractéristiques et en même temps l'une des originalités du criblage différentiel qualitatif est que cette technique aboutit à non pas une mais avantageusement deux banques différentielles qui représentent l'ensemble des différences qualitatives qui existent entre deux situations données : Paire de banque (voir figure 1 D).

Ainsi, l'invention propose toute composition ou banque d'acides nucléiques, susceptible d'être obtenue par hybridation entre une population d'ARN provenant d'un premier échantillon biologique et une population d'ADNc provenant d'un deuxième échantillon biologique. Plus préférentiellement, ces banques ou compositions comprennent des acides nucléiques représentatifs des différences qualitatives d'expression entre deux échantillons biologiques, et sont produites par un procédé comprenant (i) une étape au moins d'hybridation entre une population d'ARN provenant d'un premier échantillon biologique et une population d'ADNc provenant d'un deuxième échantillon biologique, (ii) la sélection des acides nucléiques représentatifs des différences qualitatives d'expression et, éventuellement (iii) le clonage desdits acides nucléiques.

En outre, après constitution de telles banques, il est possible d'effectuer une étape de sélection des clones pour améliorer la spécificité des banques obtenues. En effet, il est possible que certains mésappariements observés ne soient pas uniquement dus à différences qualitatives (e.g. à des épissages alternatifs différentiels), mais puissent résulter de défaut(s) de la transcription inverse par exemple. Bien que ces événements ne soient pas généralement significatifs, il est préférable de les éliminer ou de les réduire préalablement au clonage des acides nucléiques. Pour ce faire, les clones de la banque peuvent être hybridés avec les populations d'ADNc des deux situations physiologiques étudiées (voir étape (c) ci-dessus). Les clones hybridant de façon non différentielle avec les deux populations peuvent être considérés comme non-spécifiques et éventuellement éliminés ou traités en deuxième priorité (en effet, l'apparition d'une nouvelle isoforme dans l'échantillon test ne signifie pas toujours que l'isoforme initiale présente dans l'échantillon de référence a disparu de cet échantillon test). Les clones n'hybridant qu'avec une seule des deux populations ou hybridant de façon préférentielle avec l'une des populations sont considérés comme spécifiques et peuvent être sélectionnés en première priorité pour constituer des banques enrichies ou affinées.

Un affinage peut également être réalisé par hybridation et validation de clones avec des sondes provenant d'un nombre statistiquement relevant d'échantillons pathologiques.

La présente demande décrit également des banques constituées d'ADNc double brin, correspondant aux régions d'ARN spécifiques d'un épissage alternatif. Ces banques peuvent être constituées des acides nucléiques, généralement dans un vecteur de clonage ou de cultures cellulaires contenant lesdits acides nucléiques.

Le choix des ARN de départ détermine en partie les caractéristiques des banques obtenues :
- les ARN des deux situations A et B sont des ARNm ou des ARN totaux matures isolés selon les techniques connues de l'homme de l'art. Les banques sont alors des banques de criblage différentiel qualitatif dites restreintes, car restreintes aux différences qualitatives qui caractérisent les ARN matures des deux situations physiopathologiques.

- Les ARN de l'une des situations sont des ARNm ou totaux matures alors que les ARN de l'autre situation sont des ARN prémessagers, non maturés par épissage, isolés selon les techniques connues de l'homme de l'art, à partir de noyaux cellulaires. Dans ce cas les banques obtenues sont des banques de criblage différentiel dites complexes, puisque non restreintes aux différences entre ARN matures mais comprenant tout le répertoire des épissages transcrits dans une situation et éliminés dans l'autre, dont tous les introns.
- enfin, les ARN peuvent provenir d'une seule situation physiopathologique et dans ce cas le criblage différentiel implique les ARN matures et les prémessagers d'un même échantillon. Dans ce cas, les banques obtenues sont des banques de criblage différentiel qualitatif autologues. L'intérêt de telles banques est qu'elles rassemblent exclusivement le répertoire des introns transcrits dans une situation donnée. Leur hybridation avec une sonde provenant d'ARN matures d'une autre situation détermine rapidement si à cette situation est caractérisée par une rétention d'introns tout en permettant aisément leur identification.

Généralement, les banques sont générées par étalement, sur milieu solide (notamment sur milieu gélosé), d'une culture cellulaire transformée par les acides nucléiques clonés. La transformation est réalisée par toute technique connue de l'homme du métier (transfection, phosphate de calcium, électroporation, infection par des bactériophages, etc). La culture cellulaire est généralement une culture de bactéries, telles que par exemple les bactéries E. coli. Il peut également s'agir de cultures de cellules eucaryotes, notamment de cellules eucaryotes inférieures (levures par exemple). Cet étalement peut être réalisé sur boite ou sur tout autre support adapté, en conditions stériles. En outre, ces cultures étalées en milieu gélosé peuvent être stockées sous forme congelée par exemple (dans du glycérol ou autre agent adapté). Ces banques peuvent naturellement être utilisées pour la production de "répliques", c'est-à-dire de copies selon les techniques habituelles détaillées ci-après. En outre, ces banques servent généralement à préparer une banque amplifiée, c'est-à-dire une banque comprenant chaque clone sous forme amplifiée. Une banque amplifiée est préparée comme suit : à partir de la culture étalée, tous les clones cellulaires sont récupérés et sont conditionnés pour être conservés sous forme congelée ou au froid, dans tout milieu adapté. Cette banque amplifiée est avantageusement réalisée à partir de cultures de bactéries E.coli, et conservée à 4°C, en conditions stériles. Cette banque amplifiée permet la préparation et la reproduction illimitée de toute banque ultérieure contenant ces clones, sur différents supports, pour différentes applications. Une telle banque permet en outre l'isolement et la caractérisation de tout clone d'intérêt. Chacun des clones constituant les banques de l'invention est en effet un élément caractéristique d'une situation physiologique, et constitue donc une cible particulièrement intéressante pour différentes études telles que la recherche de marqueurs, la préparation d'anticorps, le diagnostic, le traitement pour transfert de gènes, etc. Ces différentes applications sont discutées plus en détail plus loin. La banque est généralement préparée comme décrit ci-dessus par étalement des cultures dans un milieu gélosé, sur un support adapté (boite de pétri par exemple). L'intérêt d'utiliser un milieu gélosé est que chaque colonie peut être séparée et individualisée. A partir de cette culture, des répliques à l'identique peuvent être préparées en quantités importantes par simple "réplique" sur tout support approprié selon les techniques de l'homme de l'art. Ainsi, la réplique peut être réalisée au moyen de filtres, membranes (nylon, nitrocellulose, etc) permettant l'accrochage des cultures. Les filtres peuvent ensuite être stockés en l'état, à 4°C par exemple, sous forme desséchée, dans tout type de conditionnement qui n'altère pas les acides nucléiques. Les filtres peuvent également être traités de manière à éliminer les cellules, protéines, etc, et à ne conserver que des composants tels que les acides nucléiques. Ces traitements peuvent comprendre notamment des protéases, des détergents, etc. Les filtres traités peuvent également être conservés dans tout dispositif ou toute condition adaptés aux acides nucléiques.

Les banques d'acides nucléiques peuvent également être préparées directement à partir des acides nucléiques, par dépôt sur des biopuces ou tout autre dispositif approprié.

L'invention concerne également toute composition ou banque comprenant des oligonucléotides spécifiques d'épissages alternatifs distinguant deux situations physiologiques. Il s'agit avantageusement d'oligonucléotides simple-brin, comprenant de 5 à 50-mères, par exemple autour de 25-mères environ.

Ces oligonucléotides sont spécifiques d'épissages alternatifs représentatifs d'une situation ou d'un type de situation physiologique. Ainsi, de tels oligonucléotides peuvent être par exemple des oligonucléotides représentatifs d'événements d'épissages alternatifs caractéristiques de situations d'apoptose. Il a en effet été décrit dans la littérature que certains épissages alternatifs étaient observés dans le cadre de situations apoptotiques. Il s'agit par exemple d'épissages dans les gènes Bclx, Bax, Fas ou Grb2 notamment. A partir des données publiées et des séquences accessibles dans la littérature et/ou sur bases de données, il est possible de créer des oligonucléotides spécifiques des formes épissées et non épissées. Ces oligonucléotides peuvent par exemple être crées selon la stratégie suivante :
(a) identification d'une protéine ou d'un événement d'épissage caractéristique d'une situation d'apoptose et de la séquence du domaine épissé. Cette identification peut être basée sur des données publiées ou par compilation de séquences accessibles sur bases de données;
(b) synthèse artificielle d'un ou plusieurs oligonucléotides correspondant à une ou plusieurs régions de ce domaine, qui permettent donc par hybridation de mettre en évidence la forme non épissée dans les ARN d'un échantillon test;
(c) synthèse artificielle d'un ou plusieurs oligonucléotides correspondant à la région de jonction entre les deux domaines séparés par le domaine épissé ; permettant ainsi, par hybridation, de mettre en évidence la forme épissée dans les ARN d'un échantillon test;
(d) reproduction des étapes (a) à (c) ci-dessus avec d'autres protéines ou événements d'épissages caractéristiques d'une situation d'apoptose;
(e) transfert sur un premier support approprié du ou des oligonucléotides spécifiques des formes apoptotiques des messagers identifiés ci-avant et, sur un autre support approprié, du ou des oligonucléotides spécifiques des formes non-apoptotiques.

Les deux supports ainsi obtenus peuvent être utilisés pour tester l'état physiologique de cellules ou échantillons tests, et notamment leur état apoptotique, par hybridation d'une préparation d'acides nucléiques de ces cellules ou échantillons.

D'autres banques similaires peuvent être générées avec des oligonucléotides spécifiques d'états physiopathologiques différents (neurodégénérescence, toxicité, prolifération, etc.) et ainsi permettre un élargissement des domaines d'applications.

Des banques d'introns ou d'exons alternatifs peuvent aussi être des banques de données informatiques constituées par analyse systématique des banques de données qui regroupent les informations relatives au génome de tel ou tel organisme, tissu ou culture cellulaire. Dans ce cas, les données obtenues par constitution de telles banques virtuelles peuvent être utilisées pour générer des amorces oligonucléotidiques qui seront utilisées pour tester en parallèle deux situations physiopathologiques.

Les données des banques informatiques peuvent également être utilisées pour dériver des sondes nucléotidiques générales, représentatives d'une classe de protéines ou encore spécifiques d'une séquence définie. Ces sondes peuvent ensuite être appliquées sur les banques de clones issues des différentes techniques de clonage des introns et exons alternatifs afin d'obtenir une image de la complexité de ces banques moléculaires et de déterminer rapidement si telle ou telle classe de protéines ou telle ou telle séquence déterminée est épissée différentiellement entre deux états physiopathologiques distincts.

### Puces à ADN

L'invention concerne également tout support (membrane, filtre, biopuce, chip, etc) comprenant une banque ou une composition d'acides nucléiques telle que définie ci-dessus. L'invention décrit également tout kit ou support comprenant plusieurs banques, notamment une banque représentative des qualités d'un état physiologique test par rapport à un état physiologique de référence et, à titre de contrôle, une banque représentative des qualités de l'état physiologique de référence par rapport à l'état physiologique test ("paire de banques"). Un kit avantageux selon l'invention comprend donc deux banques qualitatives différentielles de deux situations physiologiques (une "paire de banques"). Selon un mode de réalisation particulier, les kits de l'invention comprennent plusieurs paires de banques telles que définies ci-dessus, correspondant à différents états physiologiques ou à différents échantillons biologiques par exemple. Les kits peuvent comprendre par exemple ces différentes paires de banques déposées en série sur un même support.

### Applications

Les compositions selon l'invention peuvent être utilisées pour l'identification de molécules d'intérêt thérapeutique ou diagnostique, ou de protéines ou domaines protéiques affectés dans une pathologie.

L'un des atouts de ces techniques est en effet d'identifier à l'intérieur d'un messager, et par conséquent de la protéine correspondante, les domaines fonctionnels qui sont affectés dans une pathologie donnée. Cela permet d'assigner à un domaine donné une importance dans le développement ou le maintien d'un état pathologique. L'avantage immédiat de restreindre à un domaine précis d'une protéine l'impact d'une dérégulation pathologique est de proposer celui-ci comme une cible relevante pour un criblage de petites molécules à visée thérapeutique. Ces informations constituent également des clefs qui permettent de concevoir des polypeptides à activité thérapeutique délivrables par thérapie génique; Ces polypeptides peuvent notamment être des anticorps simples chaînes dérivés d'anticorps neutralisants dirigés contre les domaines identifiés par les techniques décrites ci avant.

Les épissages alternatifs d'exons qui différencient deux états physiopathologiques traduisent un niveau de régulation de l'expression génétique qui permet de moduler (plus précisément d'abolir ou d'instaurer) une ou plusieurs fonctions d'une protéine donnée. Ainsi la plupart des domaines structuraux et fonctionnels (SH2, SH3, PTB, PDZ, et les domaines catalytiques de différentes enzymes...) étant codés par plusieurs exons contigus, deux configurations peuvent se présenter :
i) Les domaines sont tronqués dans la situation pathologique (Zhu, Q. et al, 1994, J. Exp. Med., vol 180, n°2, pp461-470); cela indique que les chemins de signalisation impliquant ces domaines doivent être restaurés dans un but thérapeutique.
ii) Les domaines sont maintenus au cours d'une pathologie alors qu'ils sont absents dans une situation saine; ces domaines peuvent être considérés comme des cibles de criblage de petites molécules chimiques destinées à antagoniser les signaux transduits par l'intermédiaire de ces domaines.

Les séquences épissées différentiellement peuvent correspondre à des régions non-codantes situées en 5' ou en 3' de la séquence codante ou à des introns intervenant entre deux exons codants. Dans les régions non codantes, ces épissages différentiels peuvent traduire une modification de la stabilité ou de la traductibilité du messager (Bloom, T. J., and Beavo, J. A., 1995, Proc. Natl. Acad. Sci. USA, vol93, n° 24, pp 14188-14192; Ambartsumian, N. et al., 1995, Gene, vol 159, n° 1, pp 125-130). Ces phénomènes doivent alors être recherchés sur la base de ces informations et peuvent mettre en évidence que l'accumulation ou la disparition de la protéine correspondante la désigne ainsi comme cible d'intérêt. Lorsque la rétention d'un intron se produit dans une séquence codante, il s'ensuit le plus souvent une troncature de la protéine naturelle par introduction de codon stop dans la phase de lecture (Varesco, L., et al, 1994, Hum. Genet., vol 93, n°3, pp281-286; Canton, H., et al, 1996, Mol. Pharmacol., vol 50, n° 4, pp799-807, Ion, A., et al, 1996, Am. J. Hum. Genet., vol 58, n°6, pp1185-1191). Avant de rencontrer ce codon stop, il se produit généralement une lecture de quelques codons supplémentaires, ce qui aboutit à adjoindre à la partie déjà traduite une séquence spécifique, témoin protéique de l'épissage alternatif. Ces acides aminés supplémentaires peuvent être utilisés pour générer des anticorps spécifiques de la forme alternative caractéristique de la situation pathologique. Ces anticorps peuvent être ensuite utilisés comme outils de diagnostic. La protéine tronquée voit ses propriétés modifiées, voire altérées. Ainsi des enzymes peuvent être amputées de leur domaine catalytique ou de leur domaine régulateur, devenant inactives ou constitutivement activées. Des adaptateurs peuvent perdre leur capacité à connecter différents partenaires d'une cascade de signalisation (Watanabe, K. et al, 1995, J. Biol. Chem., vol 270, n°23, pp13733-13739). Les produits d'épissage des récepteurs peuvent aboutir à des récepteurs qui ont perdu leur capacité à lier leur ligand (Nakajima, T. et al, 1996, Life Sci., vol58, n°9, pp761-768) et peuvent également générer des formes de récepteurs solubles par relargage de leur domaine extracellulaire (Cheng J., 1994, Science, vol263, n° 5154, pp1759-1762). Dans ce cas, des tests diagnostiques peuvent être envisagés, basés sur la circulation dans les différents fluides physiologiques de forme soluble de récepteur à un ligand donné.

Les compositions de linvention peuvent être utilisées pour l'identification de domaines antigéniques spécifiques de protéines impliquées dans une pathologie ou pour le diagnostic de pathologies.

A titre d'exemple plus spécifique, l'invention permet avantageusement de mettre en évidence des gènes suppresseurs de tumeurs. En effet, de nombreux exemples indiquent que l'un des modes d'inactivation des gènes suppresseurs au cours de la progression tumorale est une inactivation par modulation de l'épissage de formes alternatives.

Ainsi, dans les carcinomes pulmonaires à petites cellules, le gène de la protéine p130 qui appartient à la famille RB (protéine du rétinoblastôme) est muté à un site consensus d'épissage. La conséquence de cette mutation est l'élimination de l'exon 2 et une non synthèse de protéine due à la présence d'un codon stop précoce. Cette observation a été la première à souligner l'importance des membres de la famille RB dans la tumorigénèse. De même, dans certains cancers du poumon non à petites cellules, le gène de la protéine p161NK4A, protéine qui est un inhibiteur des kinases cyclines-dépendantes cdk4 et cdk6 est muté dans un site donneur d'épissage. Le résultat de cette mutation est la production d'une protéine tronquée à demi-vie courte ce qui a pour conséquence l'accumulation des formes phosphorylées, inactives, de RB. Par ailleurs, WT1, le gène suppresseur de tumeur de Wilms, est transcrit en plusieurs ARN messagers générés par épissages alternatifs. Dans les cancers du sein, les proportions relatives des différents variants sont modifiées par rapport au tissu sain, fournissant des outils diagnostics et des pistes pour comprendre l'importance des différents domaines fonctionnels de WT1 dans la progression tumorale. Ce même phénomène de modification des rapports entre différentes formes d'ARN messagers et d'isoformes protéiques lors de la transformation cellulaire est retrouvé pour la neurofibrine NF1. En outre, cette notion de modulation des phénomènes d'épissage qui signe la progression tumorale est soutenue également par l'exemple de HDM2 dont 5 épissages alternatifs sont détectés dans les carcinomes ovariens et pancréatiques et dont les expressions augmentent selon le stade d'avancement tumoral. D'autre part, dans les cancers de la tête et du cou, l'un des mécanismes d'inactivation de p53 implique une mutation dans un site consensus d'épissage.

Les compositions de l'invention peuvent également être utilisées pour anticiper (prédire) le potentiel toxique de composés tests.

Les programmes génétiques engagés lors du traitement de cellules ou de tissus par des agents toxiques sont en grande partie corrélés aux phénomènes d'apoptose ou mort cellulaire programmée. L'importance des phénomènes d'épissage alternatif dans la régulation de ces voies apoptotiques est bien illustrée par la littérature. Cependant, aucune technologie génomique décrite jusqu'à présent ne permettait de rechercher systématiquement et d'isoler de manière exhaustive les variations de séquences dues à des épissages alternatifs et distinguant deux situations physiopathologiques données.

Les compositions de l'invention peuvent donc également être utilisées pour la détection ou le suivi du potentiel toxique et/ou thérapeutique d'un composé basée sur la détection de formes et/ou de profils d'épissages induits par ce composé sur un échantillon biologique.

Des exemples d'application de telles banques peuvent être fournis par des modèles de culture d'hépatocytes, telle la lignée HepG2, de cellules épithéliales rénales, telle la lignée HK-2, ou de cellules endothéliales, telle la lignée ECV304, traités par des agents toxiques tel l'éthanol, la camptothécine ou le PMA.

Un exemple de choix peut également être fourni par l'utilisation en cosmétologie de modèles de culture de peau traités ou non par des agents toxiques ou irritants.

Les compositions de l'invention peuvent également être utilisées pour évaluer (prédire) ou améliorer le potentiel thérapeutique de composés tests (génopharmacologie).

Dans cette utilisation, le principe mis en application est très proche de celui décrit précédemment. Des banques différentielles de référence sont établies entre les ADNc et les ARN d'une culture cellulaire ou d'un organe dans une situation contrôle et de leur équivalent mimant un modèle de pathologie. L'efficacité thérapeutique d'un produit peut alors être évaluée en suivant sa capacité à antagoniser les variations qualitatives de l'expression génique qui sont spécifiques du modèle pathologique. Cela est mis en évidence par la modification du profil d'hybridation d'une sonde issue du modèle pathologique sur les banques de références: sans traitement, la sonde n'hybride qu'avec la banque qui contient les signatures spécifiques de la maladie. Après traitement avec un produit efficace, la sonde bien que provenant du modèle pathologique hybride préférentiellement avec l'autre banque, qui porte les signatures du modèle équivalent sain.

Un exemple d'une telle application peut être fourni par un modèle d'apoptose mimant certains aspects de la neurodégénérescence qui sont antagonisés par des facteurs trophiques de référence. Ainsi les cellules dérivées de phéochromocytômes PC12 différenciées en corps neuronaux en présence de NGF entrent en apoptose par retrait de ce facteur de croissance. Cette apoptose est accompagnée par expression de nombreux marqueurs de mort cellulaire programmée dont plusieurs sont régulés par épissage alternatif et dont l'apparition est inhibée par action d'IGF1. Deux banques issues de criblage différentiel qualitatif sont établies à partir d'ARNm extraits de cellules PC12 différenciées entrées en apoptose par retrait de NGF d'une part et à partir de PC12 différenciées sauvegardées de l'apoptose par ajout d'IGF1 d'autre part. Sur ces banques peuvent être hybridées des sondes réalisées à partir d'ARNm extraits de PC12 différenciées entrées en apoptose et dont la survie est améliorée par traitement avec un produit neuroprotecteur à tester. L'efficacité de l'inversion des caractéristiques qualitatives induites par le composé test peut donc être appréciée par la capacité de la sonde à hybrider spécifiquement les clones spécifiques de la banque représentative des cellules dont la survie est améliorée. Ce test peut par la suite être utilisé pour tester l'efficacité de dérivés du composé ou de toute autre nouvelle famille de composés neuroprotecteurs et en améliorer le profil pharmacologique.

Les compositions de l'invention permettent également d'évaluer l'efficacité d'un composé test neuroprotecteur par hybridation avec une banque différentielle selon l'invention entre une cellule nerveuse saine et cette cellule présentant un modèle de neurodégénérescence.

Comme indiqué ci-avant, l'invention peut en outre être utilisée pour améliorer les propriétés d'un composé, en testant différents dérivés pour leur capacité à induire un profil d'hybridation proche de la banque représentative de l'échantillon sain.

L'invention peut aussi être utilisée en pharmacogénomique, i.e., pour évaluer (prédire) la réponse d'un patient à un composé ou traitement test.

La pharmacogénomique a pour ambition d'établir des profils génétiques de patients afin de déterminer quel traitement est susceptible d'être couronné de succès pour une pathologie donnée. Les techniques décrites dans la présente invention permettent à cet égard d'établir des banques d'ADNc représentatives des différences qualitatives qui existent entre une situation pathologique qui répond à un traitement donné et une autre qui répond peu ou mal, susceptible d'être l'objet d'une autre stratégie thérapeutique. Ces banques de références établies, elles peuvent être hybridées avec des sondes réalisées à partir d'ARN messagers de patients. Les résultats d'hybridation permettent de savoir quel patient a un profil d'hybridation correspondant à la situation de répondeur ou de non répondeur et ainsi d'affiner le choix de traitement.

Dans cette application, le but est d'une part de proposer en fonction du patient le traitement le plus approprié, le plus susceptible d'être couronné de succès et d'autre part d'enrôler dans un traitement les patients les plus susceptibles d'y répondre avec succès. Comme dans les autres applications, deux banques de criblage différentiel qualitatif sont réalisées: l'une à partir d'un modèle ou d'un échantillon pathologique connu pour répondre à un traitement, l'autre à partir d'un autre modèle ou échantillon pathologique qui répond peu ou mal à l'action thérapeutique. Ces deux banques sont ensuite hybridées avec des sondes provenant d'ARNm extraits de biopsies de différents patients. Selon que ces sondes hybrident préférentiellement avec les épissages alternatifs spécifiques de l'une ou l'autre situation, les patients peuvent être répartis en répondeurs et en non répondeurs au traitement de référence qui a défini les modèles de départ.

Ensuite, l'utilisation de filtres ou tout autre support présentant les ADNc de ces banques permet de réaliser des hybridations avec des sondes dérivées d'ARNm de biopsies de tumeurs dont on veut anticiper la réponse audit traitement. Ces résultats permettent ainsi de proposer un enrôlement optimisé des patients dans un protocole clinique.

Un exemple particulier de ce procédé consiste à déterminer la réponse de tumeurs à un traitement par le gène suppresseur de tumeur p53. Il a en effet été décrit que certains patients et certaines tumeurs répondent plus ou moins bien à ce type de traitement (Roth et al, Nature Medicine, 2 (1995) 958). Il est donc important de pouvoir déterminer quels types de tumeurs et/ou quels patients sont sensibles à un traitement par thérapie génique par p53 sauvage, afin d'optimiser le traitement et de favoriser l'enrôlement des patients dans les essais cliniques en cours. Le procédé de l'invention permet avantageusement de faciliter ces étapes en proposant des banques spécifiques des qualités de cellules répondeuses et de cellules non répondeuses à p53. Des exemples de modèles cellulaires p53-sensibles ou résistants sont décrits par exemple par Sabbatini et al. (Genes Dev. 9 (1995) 2184) ou par Roemer et al. (Oncogene 12 (1996) 2069). L'hybridation de ces banques avec des sondes dérivées de biopsies de patients permet aisément d'évaluer leur potentiel répondeur. En outre les banques spécifiques permettent également d'identifier des acides nucléiques impliqués dans la réponse à p53.

La présente demande concerne donc également l'établissement de compositions à partir d'échantillons pathologiques, ou de modèles de pathologie, qui répondent différemment à au moins un agent pharmacologique. Elle concerne aussi l'étalement de ces banques sur des filtres ou sur des supports connus de l'homme de l'art (nitrocellulose, nylon...). Avantageusement ces supports peuvent être des chips ou puces qui définissent ainsi des puces de pharmacogénomique.

Ici est décrit ainsi que des variations dans les formes et/ou profils d'épissages constituent des sources de marqueurs de pharmacogénomique, c'est-à-dire des sources de marqueurs permettant la mise en évidence de la capacité et de la manière d'un patient à répondre à des traitements. A cet égard, l'invention décrit également l'utilisation de l'intervariabilité, entre individus, des isoformes générées par épissage alternatif (analyse du spliceome) comme source de marqueurs de pharmacogénomique. Ici est décrit aussi l'utilisation de modifications d'épissage induites par des traitements comme source de marqueurs de pharmacogénomique.

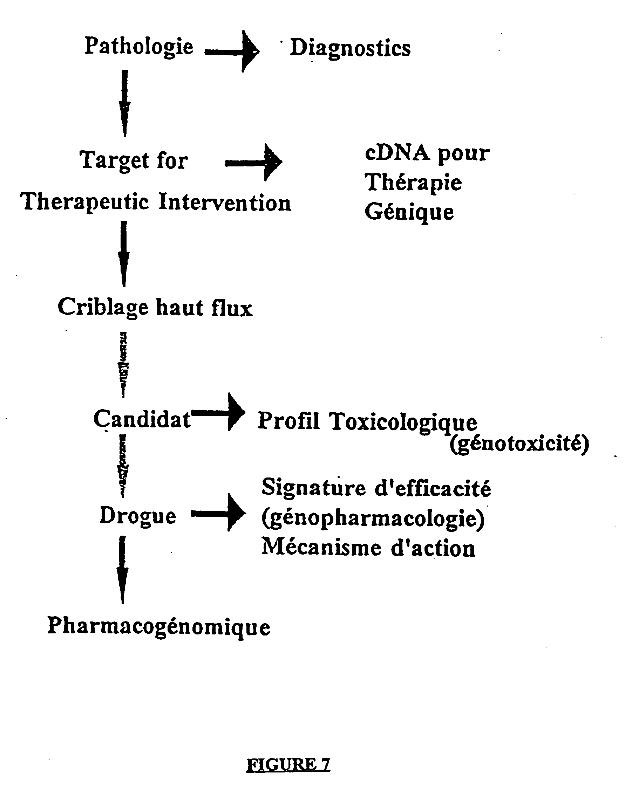

Ces différents exemples généraux illustrent l'intérêt des compositions de l'invention dans des études de génotoxicité, génopharmacologie, pharmacogénomique ainsi que dans des recherches de cibles d'intérêt diagnostique ou thérapeutique. D'autres avantages et applications de la présente invention apparaîtront à la lecture des exemples qui suivent, qui doivent être considérés comme illustratifs et non limitatifs. Les champs d'application de l'invention sont représentés sur la figure 7.

### LEGENDE DES FIGURES

Figure 1. Représentation schématique des criblages différentiels selon l'invention (figure 1A) utilisant une (figure 1B) ou deux (figure 1C) hybridations, et utilisation des acides nucléiques (figure 1 D).
Figure 2. Représentation schématique décrivant l'obtention d'hybrides ARN/ADN permettant de caractériser les séquences ARN simple brin, signatures spécifiques de l'état pathologique ou de l'état sain.
Figure 3. Représentation schématique décrivant un moyen permettant d'isoler et de caractériser par séquençage les séquences d'ARN simple brin spécifiques d'une situation pathologique ou d'une situation saine.
Figure 4. Représentation schématique décrivant un autre moyen permettant de caractériser par séquençage tout ou une partie des ARNs simple brin spécifiques d'une situation pathologique ou d'une situation saine.
Figure 5. Représentation schématique permettant d'isoler les produits d'épissages alternatifs grâce à des structures R-loop

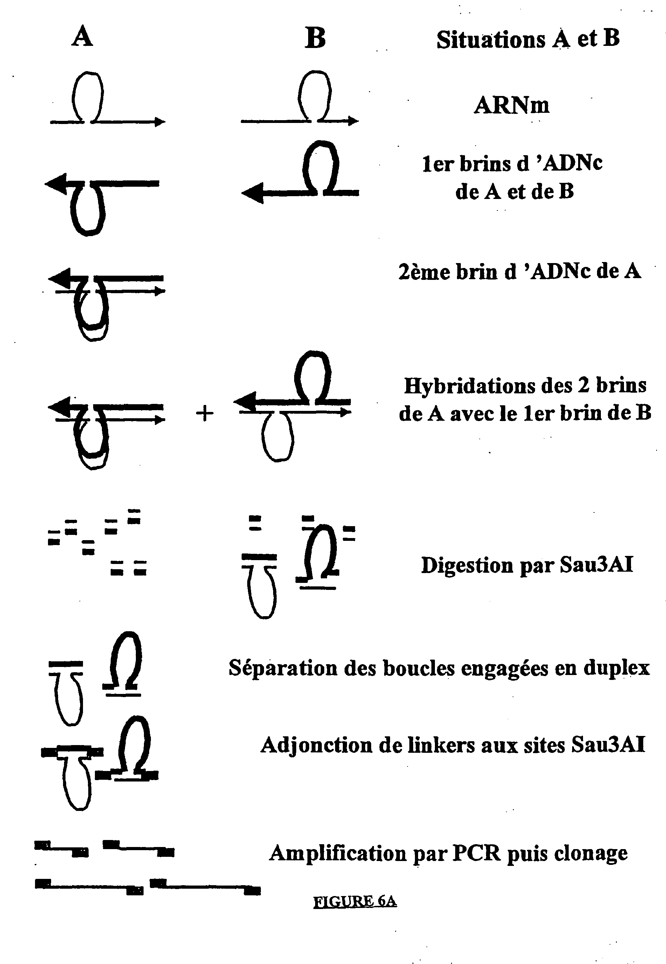

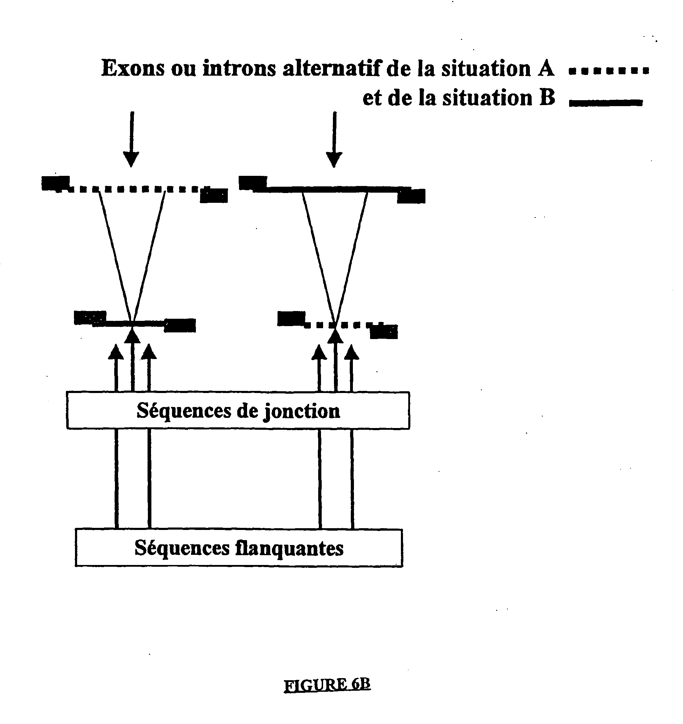

Figure 6. Représentation schématique du criblage différentiel qualitatif par restriction de boucles (formation d'homoduplexes ADNcdb/ADNc et extractions des informations, Figure 6A) et description des informations obtenues (Figure 6B).
Figure 7. Apports du criblage différentiel qualitatif aux différentes étapes de la recherche et du développement pharmaceutique.

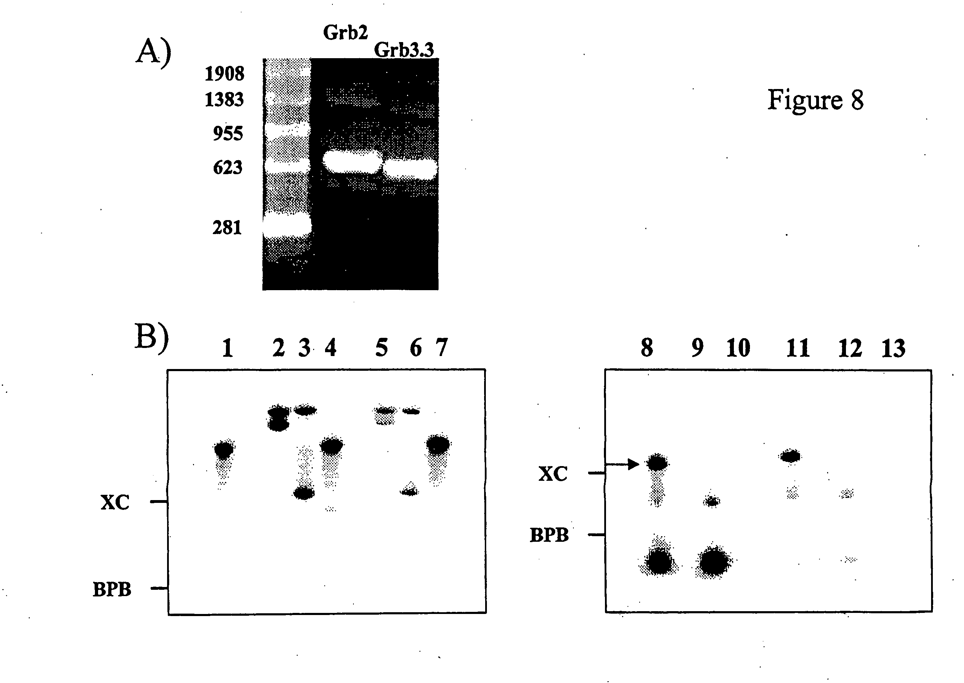

Figure 8. Isolation d'un domaine différentiellement épissé dans le modèle grb2/grb33. A) Production des ARNs synthétiques de grb2 et de grb33. B) Suivi des premières étapes de DATAS conduisant à la caractérisation d'un fragment ARN correspondant au domaine différentiellement épissé ; 1 : ARN de grb2, 2 : Hybridation entre l'ARN de grb2 et l'ADNc de grb33, 3 : Hybridation entre l'ARN de grb2 et l'ADNc de grb2, 4 : Hybridation entre l'ARN de grb2 et de l'eau, 5 : Surnageant après passage sur billes Streptavidine de (2), 6 : Surnageant après passage sur billes Streptavidine de (3), 7: Surnageant après passage sur billes Streptavidine de (4), 8 : Digestion du duplex ARN grb2 / ADNc grb33 à la Rnase H, 9 : Digestion du duplex ARN grb2 / ADNc grb2 à la Rnase H, 10 : Digestion de l'ARN grb2 à la Rnase H, 11 : pareil que (8) après passage sur colomne d'exclusion, 12 : pareil que (9) après passage sur colomne d'exclusion, 13 : pareil que (10) après passage sur colomne d'exclusion.
Figure 9. Représentation des populations d'ARNs non appariées issues de la digestion par la Rnase H à partir de duplexes ARN / ADNc simple brin provenant de cellules HepG2 traitées ou non à l'ethanol.
Figure 10. Représentation des populations d'ADNc double brin générées par une des variantes de DATAS. 1 à 12 : PCRs à partir de populations de boucles d'ARNs issues de la digestion à la Rnase H, 13 : PCR à partir d'ADNc total.
Figure 11. Application de la variante de DATAS faisant intervenir les ADNc double brin sur le modèle grb2/grb33. A) Analyse sur gel d'agarose des complexes après hybridation : 1 : ADNc double brin grb2 / ARN grb33, 2 : ADNc double brin grb2 /ARN grb2, 3 : ADNc double brin grb2 / eau. B) Digestion des échantillons 1,2 et 3 de A) par la nucléase S1 et la nucléase " Mung Bean " : 1 à 3 : complexes 1 à 3 avant traitement au glyoxal ; 4 à 6 : complexes 1 à 3 après traitement au glyoxal ; 7 à 9 : Digestions de 1 à 3 par nucléase S1 ; 10 à 12 : Digestions de 1 à 3 par nucléase Mung Bean.
Figure 12. Application de la variante de DATAS faisant intervenir les ADNc simple brin et la Rnase H sur un système de cellules HepG2 traitées ou non à l'ethanol 0,1M pendant 18 heures. Les inserts clonés ont été transférés sur membrane après electrophorèse sur gel d'agarose et soumis à hybridation à l'aide de sondes correspondant aux situations traitées (Tr) ou non (NT).
Figure 13. Mode opératoire pour évaluer le potentiel toxique d'un produit.
Figure 14. Mode opératoire pour suivre l'efficacité d'un produit.
Figure 15. Mode opératoire pour étudier la susceptibilité d'une situation pathologique à un traitement.
Figure 16. Analyse d'hybridation différentielle de clones issus de DATAS à partir d'ARNs extraits de cellules induites et d'ADNc extraits de cellules non induites. A) Utilisation de colonies bactériennes déposées et lysées sur membrane. B) Southern Blot effectué à partir d'une sélection de clones de A.
Figure 17. Séquences nucléotidique et peptidique de ΔSHC (SEQ ID NO: 9 et 10).
Figure 18. Tests de cytotoxicité et d'apoptose sur cellules HepG2 traitées A) à l'ethanol ; B) à la camptothécine ; C) au PMA.
Figure 19. Réactions de RT-PCR effectuées à partir d'ARNs extraits de cellules HepG2 traitées ou non (N.T) par l'ethanol (Eth.), la camptothécine (Camp.) et le PMA (PMA) permettant l'amplification de fragments correspondants à des domaines de MACH-a, BCL-X, FASR et beta-actine comme contrôle de normalisation.

Dans les exemples et la description de l'invention, il est fait références aux séquences de la Liste de Sequences, qui contient le texte libre suivant:
<223> OLIGO
<223> OLIGO
<223> OLIGO
<223> OLIGO
<223> OLIGO
<223> OLIGO
<223> OLIGO
<223> OLIGO
<223> OLIGO
<223> OLIGO
<223> OLIGO
<223> OLIGO

### EXEMPLES

### 1. CLONAGE DIFFÉRENTIEL DES EPISSAGES ALTERNATIFS ET AUTRES MODIFICATIONS QUALITATIVES DES ARNS EN UTILISANT DES ADNc SIMPLE-BRINS

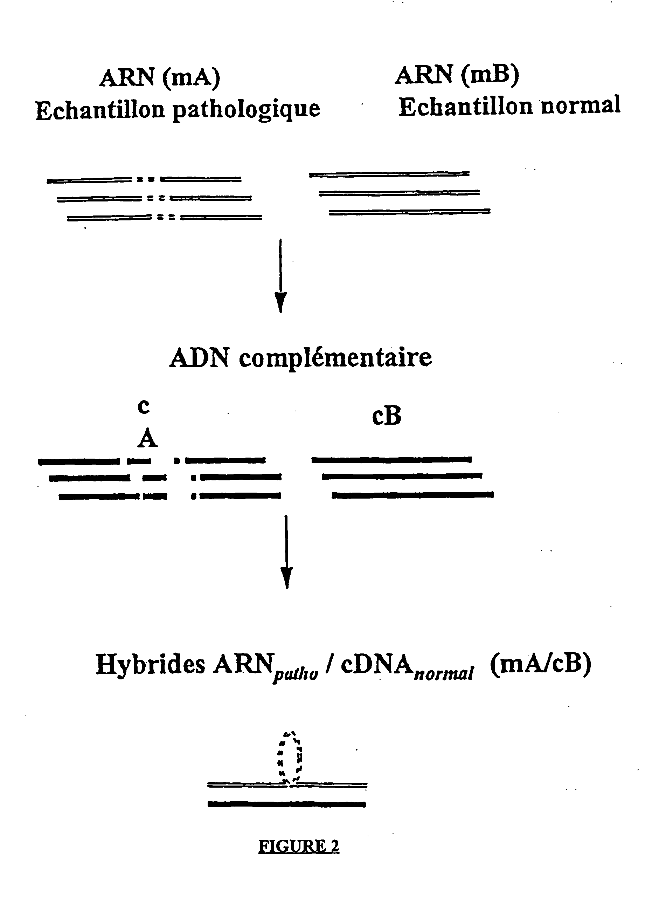

Les ARN messagers correspondant à deux situations, l'une normale (mN) et l'autre pathologique (mP), sont isolés à partir de biopsies ou de cellules en culture. Ces ARN messagers sont convertis en ADN complémentaires (cN) et (cP) à l'aide de reverse transcriptase (RT). Des hybrides mN/cP et cN/mP sont ensuite réalisés en phase liquide (se reporter au schéma de la figure 2 illustrant un des deux cas aboutissant à la formation de cN/mP).

Ces hybrides sont avantageusement réalisés en émulsion phénolique (technique PERT ou Phenol Emulsion DNA Reassociation Technique) maintenue par thermocycles (Miller, R., D. and Riblet, R., 1995, Nucleic Acids Research, vol 23, n°12, pp 2339-2340). Typiquement, cette étape d'hybridation est réalisée entre 0,1 à 1 µg d'ARN polyA+ et 0,1 à 2µg d'ADN complémentaire dans une émulsion formée d'une phase aqueuse (tampon phosphate de sodium 120mM, NaCl 2,5M, EDTA 10mM) et d'une phase organique représentant 8% de la phase aqueuse et constituée de phénol bidistillé.

Une autre technique est également avantageusement employée de façon à obtenir des hétéroduplex : à l'issue de la transcription inverse, l'ADNc néosynthétisé est séparé de l'amorce oligodT biotinylée sur colonne d'exclusion. 0,1 à 2µg de cet ADNc est coprécipité avec 0,1 à 1µg d'ARN polyA+ en présence de 0,3M d'acétate de sodium et de deux volumes d'éthanol. Ces acides nucléiques coprécipités sont repris dans 30µl d'un tampon d'hybridation qui contient 80% de formamide, 40mM de PIPES (piperazinebis(2-ethanesulfonic acid)) ph6,4, 0,4M de NaCl et 1mM d'EDTA.

Les acides nucléiques en solution sont dénaturés par chauffage 10mn à 85°C puis leur hybridation est réalisée pendant au moins 16h et jusqu'à 48h à 40°C.

L'intérêt de la technique d'hybridation en formamide est de permettre des conditions de plus forte sélectivité lors de l'appariement des brins d'ADNc et d'ARN.

A l'issue de chacune de ces deux techniques d'hybridation, nous disposons d'héteroduplex ARN/ADN dont la perfection d'appariement dépend de l'efficacité de la RT à synthétiser la longueur totale des ADNc. Demeurent également sous forme de simples brins les régions d'ARN (et d'ADN) qui correspondent aux épissages alternatifs qui différencient les deux états physiopathologiques étudiés.

Le but de la méthode est ensuite de caractériser l'information génétique portée par ces boucles d'épissage.

Pour cela, les hétéroduplex sont purifiés par capture des ADNc (amorcés avec des oligodT biotinylés) grâce à des billes portant des groupements streptavidines. Avantageusement ces billes sont de billes douées de propriétés magnétiques, ce qui permet de les séparer des ARN non engagés dans les hétéroduplex par action d'un séparateur magnétique. De telles billes et de tels séparateurs sont disponibles commercialement.

Sont isolés à ce stade de la procédure les hétéroduplex et les ADNc non engagés dans des hybridations avec des ARN. Ce matériel est ensuite soumis à l'action de la RNaseH qui va spécifiquement hydrolyser les régions d'ARN hybridées avec les ADNc. Les produits résultant de cette hydrolyse sont d'une part les ADNc et d'autre part les fragments d'ARN qui correspondent aux boucles d'épissage ou aux régions non hybridées du fait du rendement partiel de la transcriptase inverse. Les fragments d'ARN sont séparés de l'ADN par séparation magnétique selon le même mode opératoire que celui mentionné plus haut et par digestion avec de la DNase exempte de toute contamination par une activité Rnase.

### 1.1. Validation de la technique DATAS sur les variants d'épissage du gène Grb2

Une mise en évidence de la faisabilité de cette approche a été réalisée sur un système in-vitro utilisant un ARN correspondant à la région codante de Grb2 d'une part et un ADNc simple brin complémentaire à la région codante de Grb3.3. Grb2 est un gène possédant une phase codante de 651 paires de bases. Grb33 est une isoforme de grb2 générée par épissage alternatif et comprenant une délétion de 121 paires de bases dans le domaine fonctionnel SH2 de grb2 (Fath et.al, Science (1994), 264, 971-4). Les ARNs de Grb2 et de Grb33 sont synthétisés selon les techniques connues de l'homme du métier à partir d'un plasmide contenant la séquence codante de Grb2 ou de Grb33 sous contrôle du promoteur T7 à l'aide du kit RiboMax (Promega). L'analyse des produits démontre une synthèse homogène (figure 8A). Dans un but de visualisation, l'ARN de Grb2 a également été rendu radioactif par incorporation d'une base marquée lors de la transcription in-vitro à l'aide du kit RiboProbe (Promega). Les ADNc de Grb2 et de Grb3.3 ont été synthétisés par transcription inverse à partir des ARNs synthétiques produits ci-dessus, du kit Superscript II (Life Technologies) et d'une amorce oligonucleotidique biotinylée commune à Grb2 et à Grb33 correspondant au complémentaire de la séquence (618-639) de Grb2. Les ARNs et ADNcs ont été traités selon les indications des fournisseurs (promega, Life Technologies), purifiés sur colonne d'exclusion (Rnase free sephadex G25 ou G50, 5 Prime, 3 Prime) et quantifiés par spectrophotométrie.

Les premières étapes de DATAS ont été appliquées en associant, en suspension 10ng d'ARN marqué de Grb2 avec :
1. 100 ng d'ADNc de grb33 biotinylé,
2. 100 ng d'ADNc de grb2 biotinylé,
3. de l'eau
dans 30 µl d'un tampon d'hybridation qui contient 80% de formamide, 40 mM PIPES (pH 6,4), 0,4 M NaCl, 1 mM EDTA. Les acides nucléiques sont dénaturés par chauffage 10 mn à 85 °C, puis l'hybridation est réalisée pendant 16 heures à 40°C. Après capture à l'aide de billes streptavidine, les échantillons sont traités à la RNase H comme décrit précédemment.

L'analyse de ces étapes est réalisée par electrophorèse sur gel d'acrylamide à 6% suivi d'un traitement des gels à l'aide d'un Instant Imager (Packard Instruments) permettant la qualification et la quantification des espèces issues de l'ARN de grb2 marqué (figure 8B). Ainsi, les puits 2,3 et 4 indiquent que les duplexes grb2/grb33 et grb2/grb2 se sont formés de façon quantitative. La migration du complexe grb2/grb33 est retardée par rapport à celle de l'ARN de grb2 (puits 2) alors que celle du complexe grb2/grb2 est augmentée (puits 3). Les puits 5,6 et 7 correspondent aux échantillons non retenus par les billes streptavidine démontrant que 80% des complexes grb2/grb33 et grb2/grb2 ont été retenus sur les billes alors que l'ARN de grb2 seul, non biotinylé, se retrouve exclusivement dans le surnageant des billes. Le traitement à la Rnase H libère, outre les nucléotides libres qui migrent plus vite que le bleu de Bromophénol (BPB) une espèce migrant en deçà du bleu de xylène Cyanol (XC) (marqué par une flèche sur la figure) et ce spécifiquement dans le puits 8 correspondant au complexe grb2/grb33 par rapport aux puits 9 et 10 qui correspondent au complexe grb2/grb2 et à l'ARN de grb2. Les puits 11,12 et 13 correspondent aux puits 8,9 et 10 après passage des échantillons sur une colomne d'exclusion pour éliminer les nucléotides libres. La migration observée dans les puits 8 et 11 est celle attendue pour une molécule d'ARN correspondant à la délétion de 121 nucléotides différenciant grb2 de grb33.

Ce résultat montre bien la possibilité d'obtenir les boucles d'ARN générées par la formation d'hétéroduplex entre deux séquences dérivées de deux isoformes d'épissage.

### 1.2. Application de la technique DATAS à la génération de banques qualitatives de cellules hépatiques dans un état sain et toxique

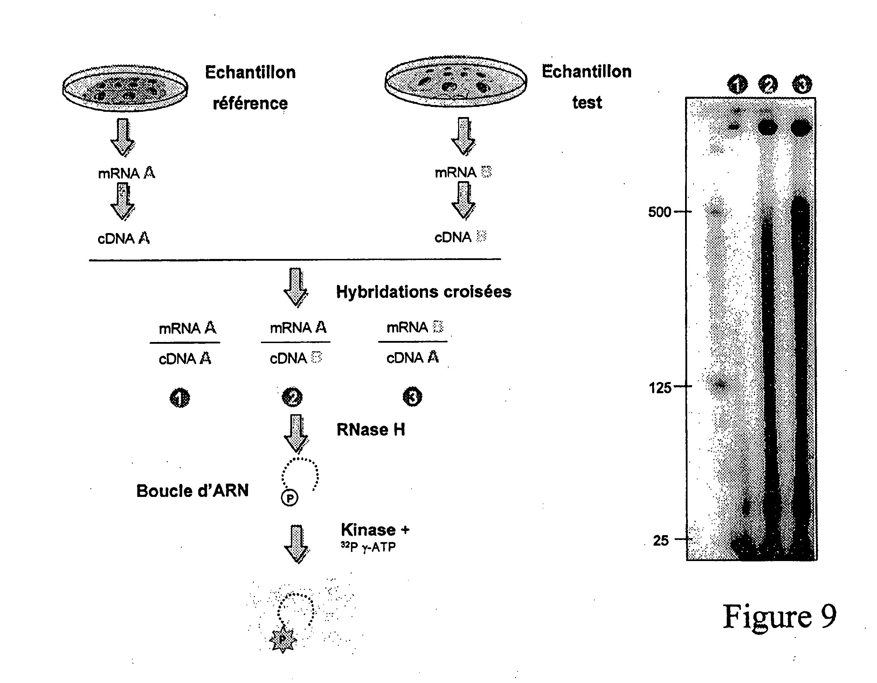

Une situation plus complexe a été étudiée. Dans le cadre de l'application de la technologie DATAS comme outil prédictif de toxicité de molécules, des cellules humaines de type hépatocytaire, HepG2, ont été traitées par de l'ethanol 0,1 M pendant 18 heures. Les ARNs ont été extraits à partir des cellules traitées ou non. La variante de DATAS décrite ci-dessus (préparation d'ADNc sb biotinylés, hybridations croisées en phase liquide, application d'un champ magnétique pour séparer les espèces, traitement RNaseH) a été appliquée avec les cellules non traitées en situation de référence (ou situation A) et les cellules traitées en situation test (ou situation B) (figure 9). Les ARNs extraits n'étant pas marqués radioactivement , la visualisation des populations d'ARN générées par digestion à la RnaseH est réalisée en effectuant une réaction d'échange du phosphate en 5' des ARNs par un phosphate marqué, à l'aide de polynucleotide kinase de T4 et de gamma-P³²ATP. Ces marquages sont ensuite déposés sur un gel d'acrylamide/urée et analysés par exposition à l'aide d'un Instant Imager (Packard Instruments). Des signatures complexes issues des hybridations A/B et B/A peuvent alors être visualisées avec un premier groupe de signaux migrant faiblement dans le gel et correspondant à des séquences d'acides nucléiques de taille importante et un deuxième groupe de signaux migrant entre 25 et 500 nucleotides. Ces signatures sont d'intensité beaucoup plus faible à partir de la situation A/A suggérant que l'ethanol peut induire une reprogrammation de l'épissage des ARNs, traduite par l'existence de signaux en A/B et B/A.

### 1.3. Clonage et Préparation de banques à partir des acides nucléiques identifiés.

Plusieurs variantes expérimentales sont ensuite envisageables pour cloner ces fragments d'ARN résistant à l'action de la Rnase H:

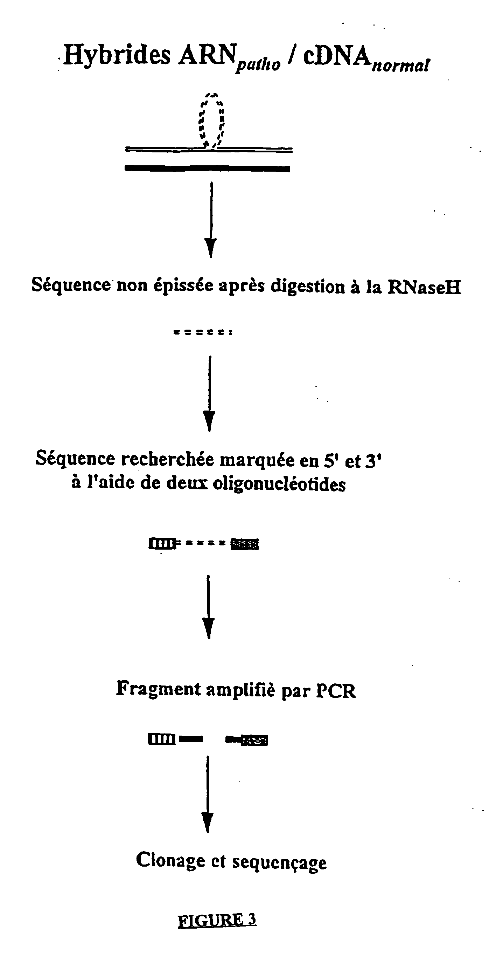

A. Une première approche consiste à isoler ces boucles et à les cloner (Figure 3).

Selon cette approche, il est procédé à une ligation d'oligonucléotides à chacune des extrémités par action de la RNA ligase selon les conditions connues de l'homme de l'art. Ces oligonucléotides sont ensuite utilisés comme amorces pour effectuer une RT PCR. Les produits de PCR sont clonés et criblés avec des sondes d'ADN complémentaires totales correspondant aux deux situations physiopathologiques d'intérêt. Seuls les clones hybridant préférentiellement avec une seule des deux sondes contiennent les boucles d'épissage qui sont ensuite séquencées et/ou utilisées pour générer des banques.

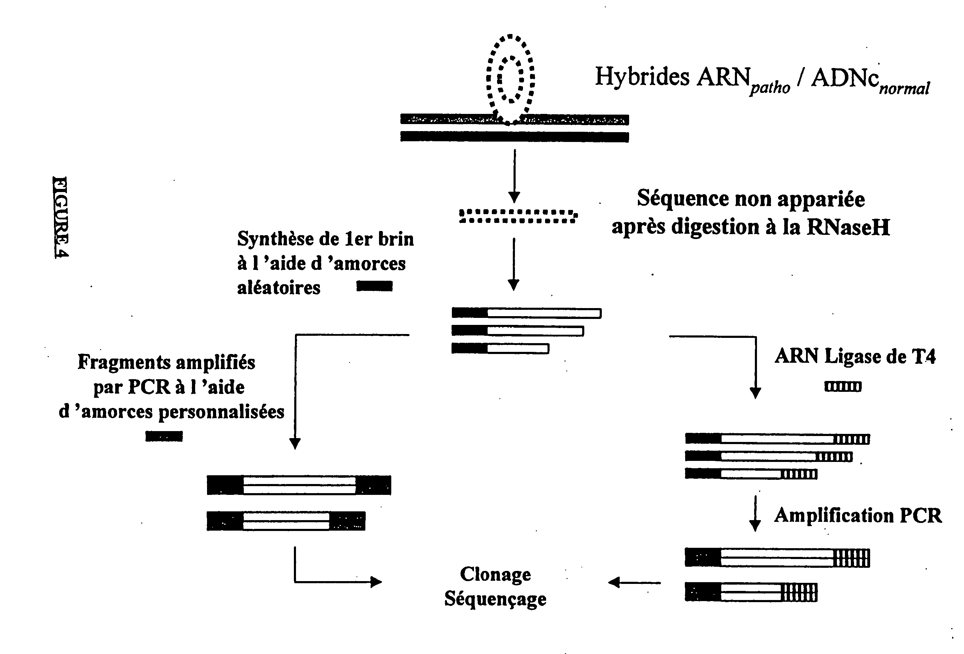

B. La seconde approche (Figure 4) consiste à effectuer une transcription inverse sur l'ARN simple brin libéré des hétéroduplex après action de la RNaseH, initiée à l'aide d'amorces au moins en partie aléatoires. Ainsi, il peut s'agir d'amorces aléatoires en 3' et en 5', d'amorces aléatoires en 3' et déterminées en 5', ou encore d'oligonucléotides semi-aléatoires, c'est-à-dire comprenant une zone de dégénérescence et une zone définie.

Selon cette stratégie, les amorces sont donc susceptibles de s'hybrider soit n'importe où sur l'ARN simple brin, soit à chaque succession de bases fixée par le choix de l'amorce semi-aléatoire. Une PCR avec des amorces correspondant aux oligonucléotides décrits ci-dessus permet ensuite d'obtenir des séquences dérivées des boucles d'épissage.

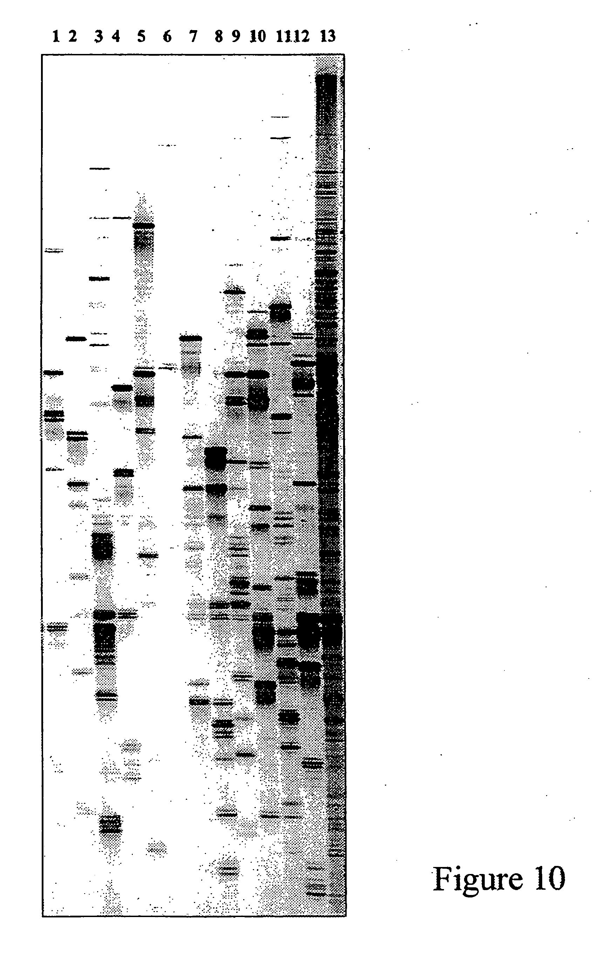

La figure 10 (puits 1 à 12) montre l'analyse sur gel d'acrylamide des fragments de PCR obtenus à partir de plusieurs essais DATAS et couplée à l'utilisation des oligonucléotides semi-aléatoires suivants:
GAGAAGCGTTATNNNNNNNAGGT (SEQ ID NO: 1, X=T)
GAGAAGCGTTATNNNNNNNAGGA (SEQ ID NO: 1, X=A)
GAGAAGCGTTATNNNNNNNAGGC (SEQ ID NO: 1, X=C)
GAGAAGCGTTATNNNNNNNAGGG (SEQ ID NO: 1, X=G)

La comparaison avec la complexité des signaux obtenus en utilisant les mêmes oligonucléotides, mais de l'ADNc total comme matrice (puits 13) démontre que DATAS a permis de filtrer ("profiler") des informations correspondant à des différences qualitatives.

Cette variante a été utilisée afin de cloner un événement correspondant au domaine ARN de grb2 généré par action de la Rnase H à partir du duplex ARN grb2 / ADNc simple brin de grb33 selon le protocole décrit précédemment (exemple 1.1.). A cette fin, un oligonucléotide de séquence : GAGAAGCGTTATNNNNNNNNTCCC (SEQ ID NO: 2), choisi sur le modèle GAGAAGCGTTATNNNNNNNWXYZ (dans lequel N est défini comme précédemment, W, X et Y représentent chacun une base fixe déterminée, et Z représente soit une base déterminée soit un groupe 3'-OH, SEQ ID NO: 3) et sélectionné pour amplifier un fragment dans la délétion de grb2 a été utilisé, permettant de générer un fragment PCR dont le clonage et le séquençage a démontré qu'il était effectivement issu du domaine délété de grb2 (194-281 dans grb2).

Ces deux approches permettent donc la production de compositions d'acides nucléiques représentatifs des épissages différentiels dans les deux situations testées qui peuvent être employées comme sondes ou pour construire des banques d'ADNc de différences qualitatives. La capacité de la technologie DATAS à générer des banques profilées d'ADNc représentatives de différences qualitatives est également illustrée par l'exemple 1.4. suivant.

### 1.4. Production de banques profilées représentatives de cellules endothéliales humaines

Cet exemple a été réalisé à partir d'une lignée de cellules endothéliales humaines (ECV304). L'analyse qualitative de l'expression génétique a été réalisée à partir d'ARN cytosoliques extraits de cellules en prolifération, d'une part, et de cellules en anoïkis (apoptose par privation de support d'attachement), d'autre part.

Les cellules ECV ont été cultivées en milieu 199 supplémenté en sels de Earle (Life Sciences). Leur mise en anoïkis a été réalisée par passage pendant 4 heures sur boîtes de culture traitées au polyHEMA. Lors de la préparation des ARN, les cellules ont été lysées dans un tampon contenant du Nonidet P-40. Les noyaux sont ensuite écartés par centrifugation. La solution d'extrait cytoplasmique a été ensuite ajustée de manière à fixer de façon spécifique l'ARN à la matrice de silice Rneasy selon les instructions de la société Qiagen. Après lavage, les ARN totaux sont élués dans de l'eau traitée au DEPC. Les ARNs messagers sont préparés à partir des ARNs totaux par séparation sur billes magnétiques Dynabeads oligo (dT)₂₅ (Dynal). Après avoir mis en suspension les billes dans un tampon de fixation, l'ARN total est incubé pendant 5 min à température ambiante. Après séparation magnétique et lavage, les billes sont reprises dans un tampon d'élution pour une incubation à 65°C qui libère les ARNs messagers.

Les synthèses d'ADN premier brin sont effectuées à partir des ARNs messagers en utilisant la Reverse Transcriptase SuperScript II ou ThermoScript (Life Technologies) à l'aide d'amorces olido (dT). Après RnaseH, les nucléotides libres sont éliminés par passage sur colonne Séphadex G50 (5 Prime- 3 Prime). Après extraction au phénol / Chloroforme et précipitation à l'éthanol, les échantillons sont quantifiés par absorbance UV.

Les quantités requises d'ARN et d'ADNc (en l'occurrence 200ng de chaque) sont combinées et précipitées à l'ethanol. Les échantillons sont repris dans un volume de 30µl dans un tampon d'hybridation (Hepes (pH 7.2) 40 mM, NaCl 400mM, EDTA 1mM) supplémenté de formamide désionisée (80% (v/v), sauf indication contraire). Après dénaturation 5 min à 70°C, les échantillons sont incubés sur la nuit à 40°C.

Les billes Streptavidine (Dynal) sont lavées puis reconditionnées dans un tampon de fixation (2X= Tris-HCl (pH 7,5) 10 mM, NaCl 2M, EDTA 1 mM). Les échantillons d'hybridation sont amenés à un volume de 200 µl avec de l'eau puis ajustés à 200 µl de billes pour une incubation de 60 min à 30°C. Après capture sur aimant et lavages des billes, celles-ci sont reprises dans 150 µl de tampon RnaseH puis incubées pendant 20 min à 37°C. Après capture sur aimant, les régions non hybridées ont été relarguées dans le surnageant qui est traité à la Dnase puis extrait au phénol acide / chloroforme puis précipité à l'éthanol. Les précipitations à l'éthanol de faibles quantités d'acides nucléiques sont effectuées à l'aide d'un polymère commercial SeeDNA (Amersham Pharmacia Biotech) permettant de récupérer de façon quantitative des acides nucléiques à partir de solutions très diluées (de l'ordre du ng/ml).

La synthèse d'ADNc à partir des échantillons d'ARNs provenant de l'action de la RnaseH est effectuée à partir d'hexanucléotides aléatoires à l'aide de Superscript II Reverse Transcriptase. L'ARN est ensuite dégradé à l'aide d'un mélange de RnaseH et de Rnase T1. L'amorce, les nucléotides non incorporés et les enzymes sont séparés de l'ADNc à l'aide d'une cartouche " GIassMAX Spin ". L'ADNc correspondant aux boucles d'épissage est ensuite soumis à une réaction de PCR en utilisant des oligonucléotides de type semi-aléatoire déjà décrits plus haut dans l'invention. En l'occurrence les oligonucléotides choisis sont :
GAGAAGCGTTATNNNNNCCA (SEQ ID NO: 4)

La réaction de PCR est réalisée à l'aide de Taq Polymérase sur 30 cycles :
- Dénaturation initiale : 94°C pdt 1 min.
- 94°C pdt 30 s
- 55°C pdt 30s
- 72°C pdt 30s
- Extension finale : 72°C pdt 5 min.

Les produits de PCR ont été clonés dans le vecteur pGEM-T (Proméga) possédant un T flottant aux extrémités 3' afin de faciliter le clonage de fragments issus de l'activité de la Taq Polymérase. Après transformation dans les bactéries JM109 compétentes (Proméga), les colonies obtenues sont repiquées sur filtre de nitrocellulose, et hybridées avec des sondes dérivées de produits de PCR effectuées sur des ADNc totaux des cellules en prolifération d'une part et en anoïkis d'autre part. Pour ces PCR les mêmes oligonucléotides GAGAAGCGTTATNNNNNCCA sont utilisés. Dans une première réalisation expérimentale, 34 clones hybridant préférentiellement avec la sonde des cellules en apoptose et 13 clones hybridant préférentiellement avec la sonde des cellules en prolifération ont été isolés.

Parmi ces 13 clones, 3 clones contiennent le même fragment d'ADNc qui dérive du domaine SH2 de la protéine SHC.

La séquence de ce fragment est la suivante :

L'utilisation d'amorces de PCR qui encadrent le domaine SH2 de SHC (oligo5' : GGGACCTGTTTGACATGAAGCCC (SEQ ID NO: 6) ; oligo3': CAGTTTCCGCTCCACAGGTTGC (SEQ ID NO: 7)) a permis de caractériser la délétion du domaine SH2 de SHC qui est observée spécifiquement dans les cellules ECV en anoïkis. Avec ce couple d'amorce, un seul produit d'amplification correspondant à un fragment d'ADNc de 382 paires de bases qui contient le domaine SH2 intègre est obtenu à partir d'ARN de cellules ECV en phase exponentielle. Un fragment additionnel de 287 paires de bases est observé lorsque la PCR est réalisée à partir d'ARN de cellules en anoïkis. Ce fragment supplémentaire dérive d'un ARN messager dérivé du messager de SHC mais présentant une délétion.

La séquence de cette délétion est la suivante :

Cette délétion correspond aux bases 1198 à 1293 de la phase ouverte du messager codant pour les formes de 52kDa et 46kDa de la protéine SHC (Pelicci, G. et al, 1992, Cell, 70, pp93-104).

Les données structurales des domaines SH2 ainsi que la littérature indiquent qu'une telle délétion aboutit à la perte de l'affinité pour les phosphotyrosines puisqu'elle englobe les acides aminés impliqués dans les interactions avec les tyrosines phosphorylées (Waksman, G. et al, 1992, Nature, vol358, pp646-653). Les protéines SHC étant des adapteurs qui connectent différents partenaires par leurs domaines SH2 et PTB (PhosphoTyrosine Binding domain), cette délétion génère donc un dominant négatif naturel de SHC que nous appelons ΔSHC. Les domaines SH2 des protéines dont les gènes sont séquencés étant portés par deux exons, il est vraisemblable que la délétion identifiée par la méthode DATAS correspond à un exon alternatif du gène SHC.

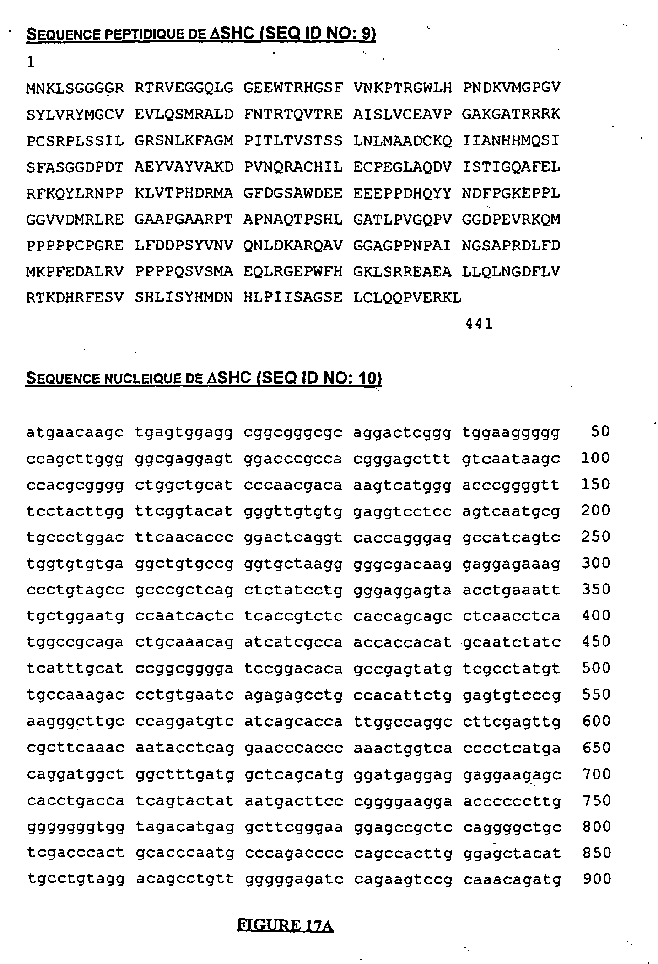

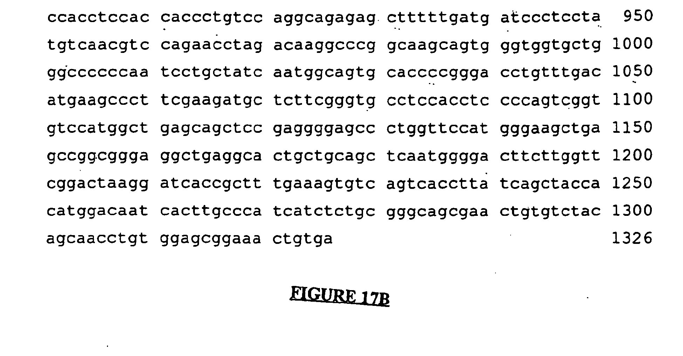

Les séquences protéique et nucléique de ΔSHC sont les représentées sur la Figure 17 (SEQ ID NO: 9 et 10).

Le domaine SH2 de SHC étant impliqué dans la transduction de nombreux signaux impliqués dans la prolifération et la viabilité cellulaires, l'examen de la séquence de ΔSHC permet d'anticiper ses propriétés de dominant négatif sur la protéine SHC et sa capacité d'interférer avec différent signaux cellulaires.

Ici est décrit également cette nouvelle forme épissée de SHC, le domaine protéique correspondant à l'épissage, tout anticorps ou sonde nucléique permettant sa détection dans un échantillon biologique, et leurs utilisation diagnostique ou thérapeutique, par exemple.

Ici est décrit en particulier tout variant de SHC comprenant au moins une délétion correspondant aux bases 1198 à 1293, plus particulièrement une délétion de la séquence SEQ ID NO: 8. L'invention concerne plus spécifiquement le variant ΔSHC ayant la séquence SEQ ID NO: 9, codé par la séquence SEQ ID NO: 10.

Ici est décrit aussi toute sonde nucléique, oligonucléotide ou anticorps permettant d'identifier le variant ΔSHC ci-dessus, et/ou toute altération du rapport SHC/ΔSHC dans un échantillon biologique. II peut s'agir notamment d'une sonde ou oligonucléotide complémentaire de tout ou partie de la séquence SEQ ID NO: 8, ou d'un anticorps dirigé contre le domaine protéique codé par cette séquence. De telles sondes, oligonucléotides ou anticorps permettent de détecter la présence de la forme non épissée (e.g, SHC) dans un échantillon biologique.

Les matériels peuvent en outre être utilisés en parallèle avec des sondes, oligonucléotides et/ou anticorps spécifiques de la forme épissée (e.g, ΔSHC), c'est-à-dire correspondant par exemple à la région de jonction résultant de l'épissage (localisée autour du nucléotide 1198 de la séquence SEQ ID NO: 10).

De tels matériels peuvent être utilisés pour le diagnostic de pathologies liées à une immunodépression (cancer, traitement immunosuppresseur, SIDA, etc.).

Ici est décrit aussi tout procédé de criblage de molécules basé sur le blocage (i) du domaine épissé dans la protéine SHC (notamment pour induire un état de tolérance immunitaire par exemple dans les maladies autoimmunes ou les rejets de greffes et les cancers) ou (ii) des gains de fonction acquis par la protéine ΔSHC.

Ici est décrit en outre l'utilisation thérapeutique de ΔSHC, et notamment pour le traitement de cellules cancéreuses ou de cancers (ex vivo ou in vivo) dans lesquels une hyperphosphorylation de la protéine SHC peut être mise en évidence, par exemple. A cet égard, Ici est décrit aussi tout vecteur, notamment viral, comprenant une séquence codant pour ΔSHC. Il s'agit préférentiellement d'un vecteur capable de transfecter des cellules cancéreuses ou en prolifération, telles que des cellules musculaires lisses, des cellules endothéliales (resténose), des fibroblastes (fibroses), de préférence d'origine mammifère, notamment humaine. Comme vecteur viral, on peut citer notamment des vecteurs adénoviraux, rétroviraux, AAV, herpès, etc.

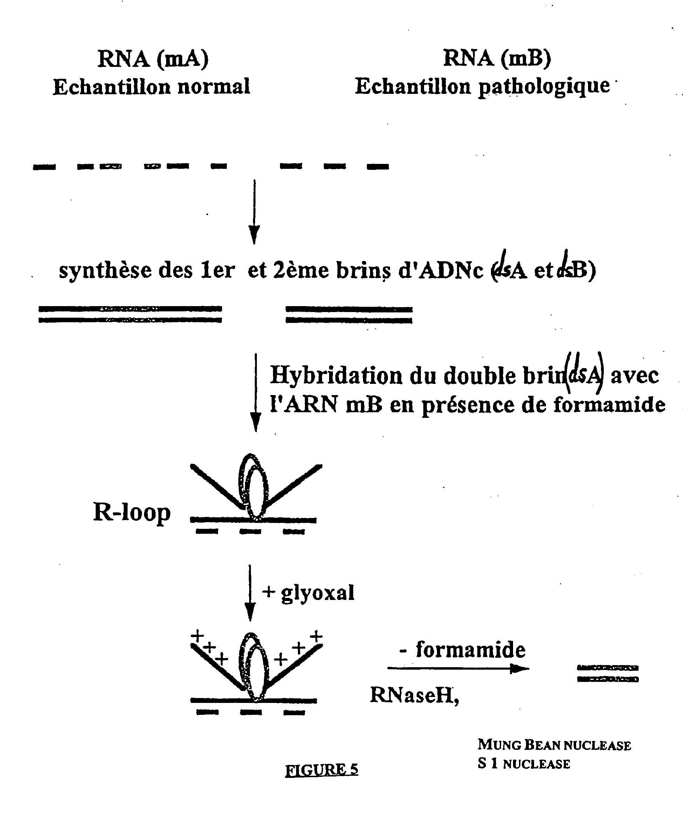

### 2. CLONAGE DIFFÉRENTIEL DES EPISSAGES ALTERNATIFS ET AUTRES MODIFICATIONS QUALITATIVES DES ARNS EN UTILISANT DES ADNc DOUBLE-BRINS (FIGURE 5).

Les ARNs messagers correspondants aux situations normales (mN) et pathologiques (mP) sont produits, ainsi que les ADNs complémentaires double brin correspondants (dsN et dsP) par des protocoles classiques de biologie moléculaire. Des structures de type "R-loop" sont alors obtenues en hybridant mN avec dsP et mP avec dsN dans une solution contenant 70% de formamide. Les domaines nucléiques différentiellement épissés entre la situation N et P resteront sous forme d'ADN double brin. Les simples brins d'ADN déplacés sont alors traités au glyoxal afin d'éviter le redéplacement du brin d'ARN lors du retrait de la formamide. Après retrait de la formamide et du glyoxal puis traitement à la RNAseH, nous nous retrouvons avec des structures de type abeille, les ADNs simples brin non appariés représentant les ailes de l'abeille et le domaine double brin appareillé d'intérêt représentant le corps de l'abeille. L'utilisation d'enzymes qui dégradent spécifiquement l'ADN simple brin comme la nucléase S1 ou la Mung Bean nucléase permet l'isolation de l'ADN resté sous forme double brin qui est ensuite cloné puis séquencé. Cette deuxième technique permet l'obtention directe d'une empreinte ADN double brin du domaine d'intérêt comparativement au premier protocole qui produit une empreinte ARN de ce domaine.

Cette approche a été réalisée sur le modèle grb2/grb33 décrit précédemment. L'ADN double brin de grb2 a été produit par amplification PCR à partir de l'ADNc simple brin de grb2 et de deux amorces nucléotidiques correspondant à la séquence (1-22) de grb2 et à la séquence complémentaire de (618-639) de grb2. Ce fragment PCR a été purifié sur gel d'agarose, nettoyé sur colonne d'affinité (JetQuick, Genomed) et quantifié par spectrophotométrie. Dans le même temps, deux ARNs synthétiques correspondant aux phases de lecture de grb2 et de grb33 ont été produits à partir de vecteurs plasmidiques comportant les cDNAs de grb2 ou de grb33 sous contrôle du promoteur T7, à l'aide du kit RiboMax (Promega). Les ARNs ont été purifiés selon les instructions du fournisseur et nettoyés sur colomne d'exclusion (Sephadex G50, 5 prime-3 prime). 600 ng de l'ADN double brin de grb2 (1-639) ont été associés avec :
1. 3 µg d'ARN de grb33
2. 3 µg d'ARN de grb2
3. de l'eau
dans trois réactions différentes, dans le tampon suivant :
100 mM PIPES (pH 7,2), 35 mM NaCl, 10 mM EDTA, 70% formamide déionisé (Sigma)

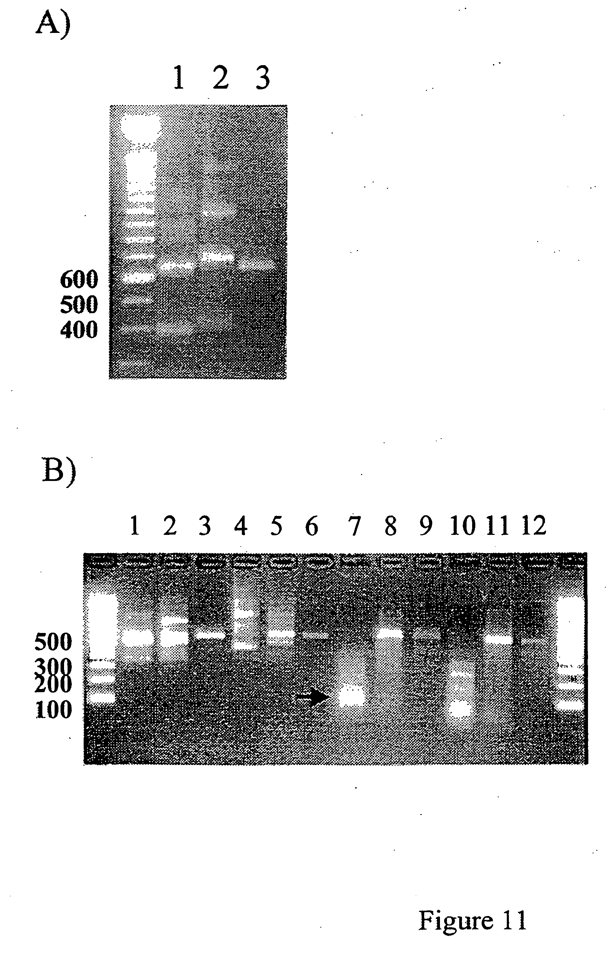

Les échantillons ont été amenés à 56 °C puis refroidis à 44 °C par incrément de -0,2 °C toutes les 10 minutes. Ils sont ensuite conservés à 4 °C. L'analyse sur gel d'agarose révèle des modifications de migrations dans les puits 1 et 2 par rapport au puits 3 contrôle (Figure 11A) indiquant la formation de nouveaux complexes. Les échantillons sont ensuite traités au glyoxal déionisé (Sigma) (5% v/v ou 1M) pendant 2 h à 12 °C. Les complexes sont ensuite précipités à l'éthanol (0,1M NaCl, 2 volumes d'ethanol), lavés à l'ethanol 70%, séchés puis repris dans de l'eau. Ils sont enfin traités par la RnaseH (Life Technologies), puis par une enzyme capable de dégrader spécifiquement l'ADN simple brin. La nuclease S1 et la nucléase " Mung Bean " présentent cette propriété et sont disponibles commercialement (Life Technologies, Amersham). De telles digestions (incubations de 5 minutes dans les tampons fournis avec les enzymes) ont été analysées sur gel d'agarose (figure 11 B). Des digestions significatives sont uniquement obtenues à partir des complexes issus de la réaction 1 (grb2/grb33) (figure 11B, puits 7 et 10). Ces digestions semblent plus complètes avec la nucléase S1 (puits 7) qu'avec la nucléase " Mung Bean " (puits 10). Ainsi, la bande correspondant à une taille légèrement supérieure à 100 paires de bases (indiquée par une flèche dans le puits 7) a été purifiée, clonée dans le vecteur pMos-Blue (Amersham), puis séquencée. Ce fragment correspond au domaine de 120 paires de bases de grb2, délété dans grb33.

Cette approche peut maintenant être effectuée à partir d'une population totale d'ARN messager et d'une population totale d'ADNc double brin produite selon les techniques connues de l'homme de métier. La population d'ARN de la situation de référence est hybridée à la population d'ADNc double brin de la situation test et réciproquement. Après application du protocole décrit ci-dessus, les digestions sont déposées sur gel d'agarose afin d'isoler et de purifier les bandes correspondant à des tailles variant entre 50 et 300 paires de bases. Ces bandes sont ensuite clonées dans un vecteur (pMos-Blue, Amersham) pour donner lieu à une banque d'inserts enrichis en des événements de différences qualitatives.

### 3. CONSTRUCTION DE BANQUES ISSUES DE CRIBLAGES DIFFÉRENTIELS QUALITATIFS.

Les deux exemples décrits ci-dessus aboutissent aux clonages d'ADNc représentatifs de toute ou partie des séquences épissées différentiellement entre deux situations physiopathologiques données. Ces ADNc permettent la constitution de banques par insertion de ces ADNc dans des vecteurs plasmidiques ou phagiques. Ces banques peuvent être présentées sur des filtres de nitrocellulose ou tout autre support connu de l'homme de l'art, tels des chips ou biopuces ou membranes. Ces banques peuvent être conservées au froid, à l'abri de la lumière. Ces banques, une fois déposées et fixées sur support par les techniques classiques, peuvent être traitées par des composés pour éliminer les bactéries hôtes qui permettent la production des plasmides ou des phages. Ces banques peuvent également avantageusement être constituées de fragments d'ADNc correspondant aux ADNc clonés mais préparés par PCR de façon à ne déposer sur filtre que les séquences dérivées des événements d'épissages alternatifs.

L'une des caractéristiques et en même temps l'une des originalités du criblage différentiel qualitatif est que cette technique aboutit de façon avantageuse non pas à une mais à deux banques différentielles ("paire de banque") qui représentent l'ensemble des différences qualitatives qui existent entre deux situations données. En particulier, l'une des banques d'épissage différentiel de l'invention représente la signature des qualités de la situation physiologique test par rapport à la situation physiologique de référence, et l'autre banque représente la signature des qualités de la situation physiologique de référence par rapport à la situation physiologique test. Ce couple de banques est également désigné paire de banques ou "banque différentielle d'épissage".

L'un des apports du criblage différentiel qualitatif étant de permettre d'évaluer le potentiel toxique d'un composé, comme cela est indiqué dans le chapitre suivant, un bon exemple de mise en oeuvre de la technologie est l'obtention par DATAS de clones d'ADNc correspondant à des séquences spécifiques de cellules HepG2 naïves, d'une part, et traitées par de l'éthanol, d'autre part. Ces cellules présentent des signes de cytotoxicité et une dégradation de leur ADN par fragmentation internucléosomale à partir de 18h en présence de 1 M d'éthanol. De façon à obtenir des marqueurs précoces de la toxicité éthanolique, les ARN messagers ont été préparés à partir de cellules naïves et de cellules traitées pendant 18h par de l'éthanol à la concentration de 0,1M. Après mise en oeuvre de la variante de DATAS qui utilise l'ADNc simple brin et la Rnase H, les ADNc clonés obtenus ont été amplifiés par PCR, soumis à une électrophorèse sur gel d'agarose et ensuite transférés sur un filtre de nylon selon les techniques connues de l'homme de l'art. Pour chaque ensemble de clones spécifiques d'une part des différences qualitatives spécifiques de l'état naïf et d'autre part des séquences spécifiques des cellules traitées par l'éthanol, deux répliques identiques de filtres sont effectuées. Ainsi les empreintes de chaque ensemble de clones sont hybridées d'une part avec une sonde spécifique des cellules non traitées et d'autre part avec une sonde spécifique des cellules traitées par 0,1 M d'éthanol pendant 18h.

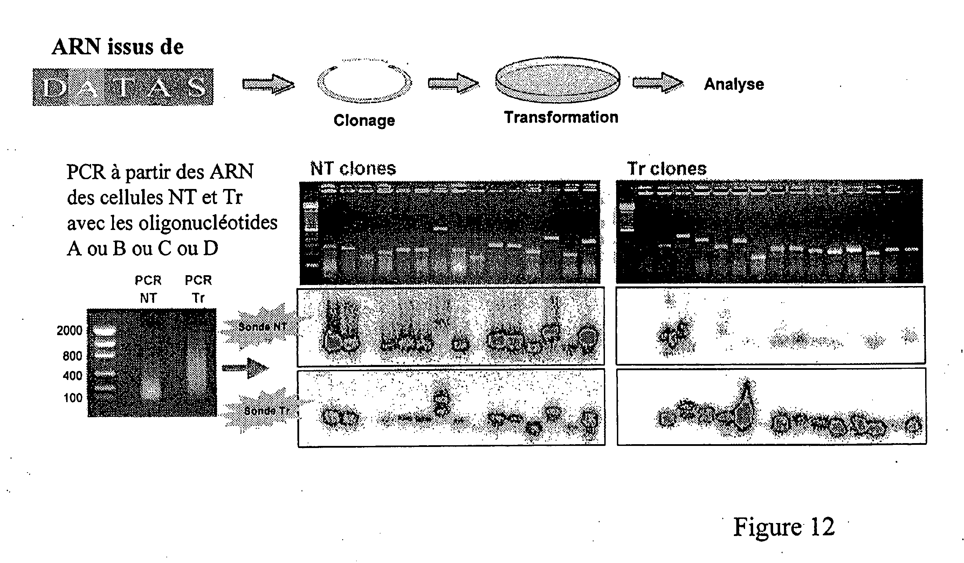

Le profil d'hybridation différentiel obtenu et présenté sur la figure 12 permet d'apprécier la qualité de la soustraction effectuée lors de la mise en oeuvre de la technique DATAS. Ainsi les clones issus de l'hybridation de l'ARNm de cellules non traitées (NT) avec l'ADNc de cellules traitées (Tr) et qui doivent correspondre à des différences qualitatives spécifiques de la situation naïve hybrident préférentiellement avec une sonde représentant la population totale des ARN messagers des cellules non traitées. Réciproquement, les clones issus des produits résistant à la RNase H ayant agi sur les hétéroduplex ARN(Tr)/ADNc(NT) hybrident préférentiellement avec une sonde dérivée de la population totale des ARN messagers des cellules traitées.

Les deux ensembles de clones spécifiques d'une part de la situation traitée et d'autre part de la situation non traitée représentent un exemple de banques de différences qualitatives caractéristiques de deux états cellulaires distincts.

### 4. UTILISATIONS ET APPORTS DES BANQUES DIFFÉRENTIELLES QUALITATIVES.

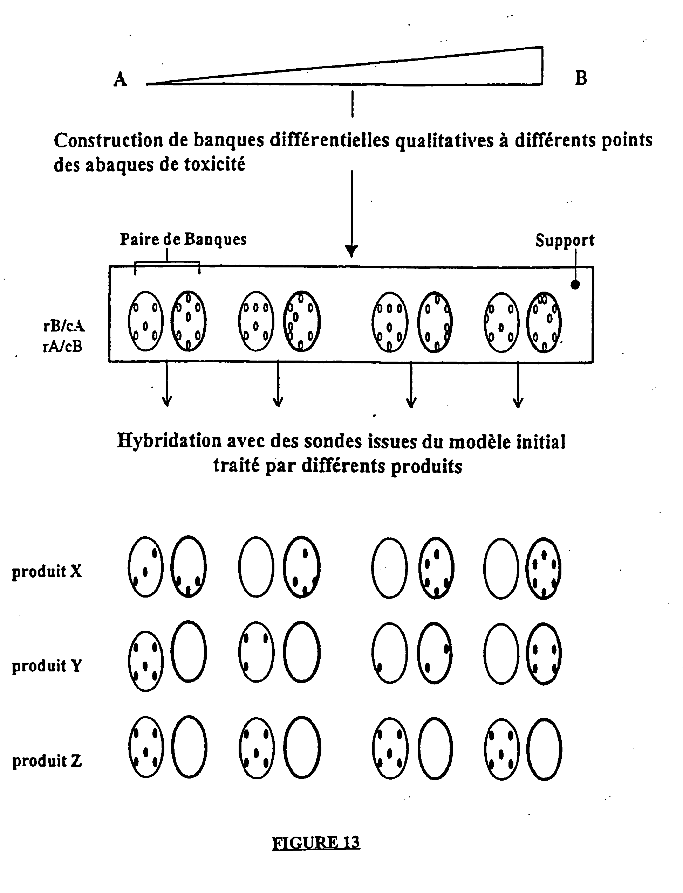

Les possibilités d'utilisation des banques différentielles d'épissage de l'invention sont illustrées notamment sur les Figures 13 à 15. Ainsi, ces banques sont utilisables pour :

### 4.1. L'évaluation du potentiel toxique d'un composé (figure 13) :

Dans cet exemple, la situation de référence est désignée A et la situation toxique est désignée B. Des abaques de toxicité sont obtenues par traitement de la situation A en présence de différentes concentrations d'un composé toxique de référence, pendant des périodes variables. A différents points les abaques de toxicité, des banques différentielles qualitatives sont construites (paires de banque), dans cet exemple, des banques restreintes rA/cB et rB/cA. Les paires de banque sont avantageusement déposées sur un support. Le support est ensuite hybridé avec des sondes issues de l'échantillon biologique initial traité par différentes doses de composés test : Produits X, Y et Z. L'hybridation est révélée et fait apparaître le potentiel toxique des produits test : dans cet exemple, le produit Z présente une forte toxicité et le produit Y offre un profil intermédiaire. La faisabilité de cette constitution d'abaques de toxicité est bien illustrée par l'exemple de constitution de banques de criblage différentiel qualitatif décrit ci-avant et mettant en jeu l'éthanol et des cellules HepG2.

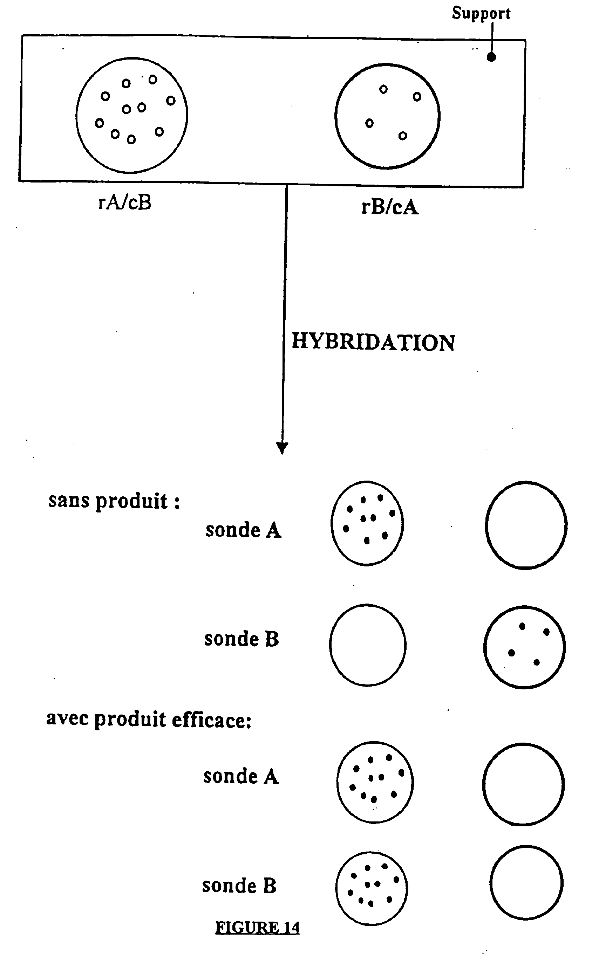

### 4.2. L'évaluation de l'efficacité d'une composition pharmaceutique (figure 14) :

Dans cet exemple, une paire de banques restreintes selon l'invention est réalisée à partir d'un modèle pathologique B et d'un modèle sain A (ou du modèle pathologique traité avec un produit actif de référence). Les banques différentielles rA/cB et rB/cA sont, le cas échéant, déposées sur un support. Cette paire de banque regroupe les différences d'épissage entre les deux situations. Cette paire de banque permet d'évaluer l'efficacité d'un composé test, c'est-à-dire de déterminer sa capacité à générer un profil de type "sain" (rA/cB) à partir du profil de type pathologique (rB/cA). Dans cet exemple, la paire de banque est hybridée avec des sondes préparées à partir des situations A et B avec ou sans traitement par le composé test. Le profil d'hybridation qui peut être obtenu est présenté sur la figure 14. La faisabilité de cette application est la même que celle de la constitution de banques de différences qualitatives caractéristiques de situations saines et toxiques présentée plus-haut. La situation toxique est remplacée par l'état pathologique et il est possible d'apprécier la capacité d'un composé test à produire une sonde hybridant avec plus ou moins de préférence avec les situations de référence ou pathologique.

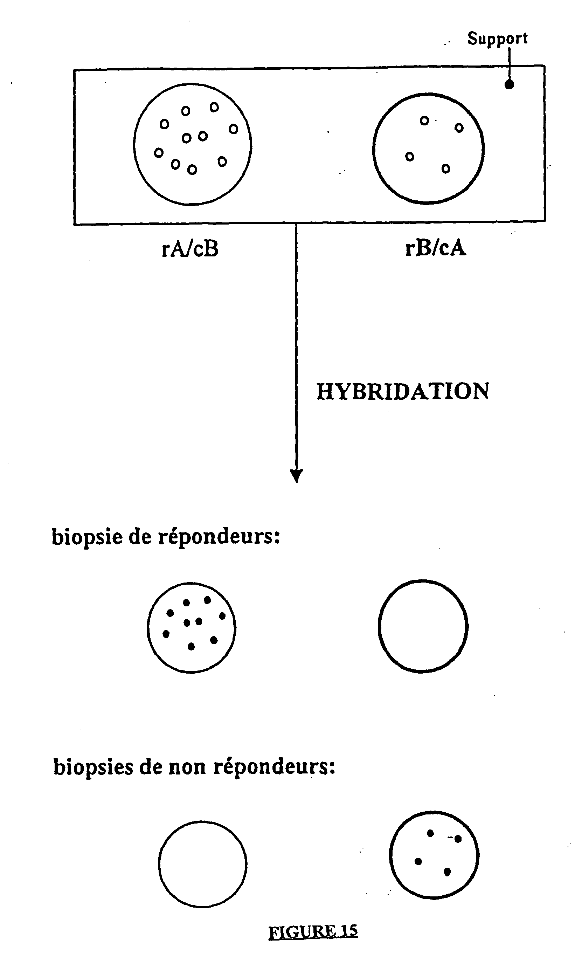

### 4.3. L'anticipation de la réponse d'un échantillon pathologique à un traitement (figure 15) :

Dans cet exemple, une paire de banque restreinte selon l'invention est réalisée à partir de deux modèles pathologiques, dont l'un répond à un traitement par un produit donné (le gène p53 sauvage par exemple) : situation A ; et l'autre y est réfractaire : situation B. Cette paire de banque (rA/cB ; rB/cA) est déposée sur un support.

Cette paire de banque est ensuite utilisée pour déterminer la sensibilité d'un échantillon pathologique test à ce même produit. Pour cela, cette paire de banque est hybridée avec des sondes provenant de biopsies de patients dont on souhaite anticiper la réponse au traitement de référence. Le profil d'hybridation d'une biopsie de répondeur et d'une biopsie de non-répondeur est présenté sur la figure 15.

### 4.4 L'identification de ligands pour des récepteurs orphelins

L'activation de récepteurs membranaires ou nucléaires par leurs ligands pourrait induire spécifiquement des dysrégulations dans l'épissage de certains ARNs. L'identification de ces événements par les méthodes DATAS de l'invention permet de disposer d'un outil (marqueurs, banques, kits, etc.) de suivi d'activation de récepteurs, utilisables pour la recherche de ligands naturels ou synthétiques de récepteurs, en particulier orphelins. Selon cette application, des marqueurs associés aux dysrégulations sont identifiés et déposés sur des supports. L'ARN total de cellules, (sur)exprimant le récepteur à l'étude, traitées ou non par différentes compositions et/ou composés tests, est extrait et utilisé comme sonde dans une hybridation avec les supports. La mise en évidence d'une hybridation avec certains, voire la totalité des marqueurs déposés sur le support, indique que le récepteur à l'étude a été activé, et donc que le composition/composé correspondant constitue ou comprend un ligand dudit récepteur.

### 4.5 L'identification de cibles d'intérêt thérapeutique :

Celle-ci se fait par identification de gènes dont l'épissage est modifié dans une pathologie ou dans un modèle de pathologie et plus précisément l'identification des exons ou introns modifiés. Cette approche doit permettre de donner accès aux séquences qui codent les domaines fonctionnels altérés lors de pathologies ou de tout phénomène physiopathologique adressant les phénomènes de prolifération, différenciation ou apoptose par exemple.

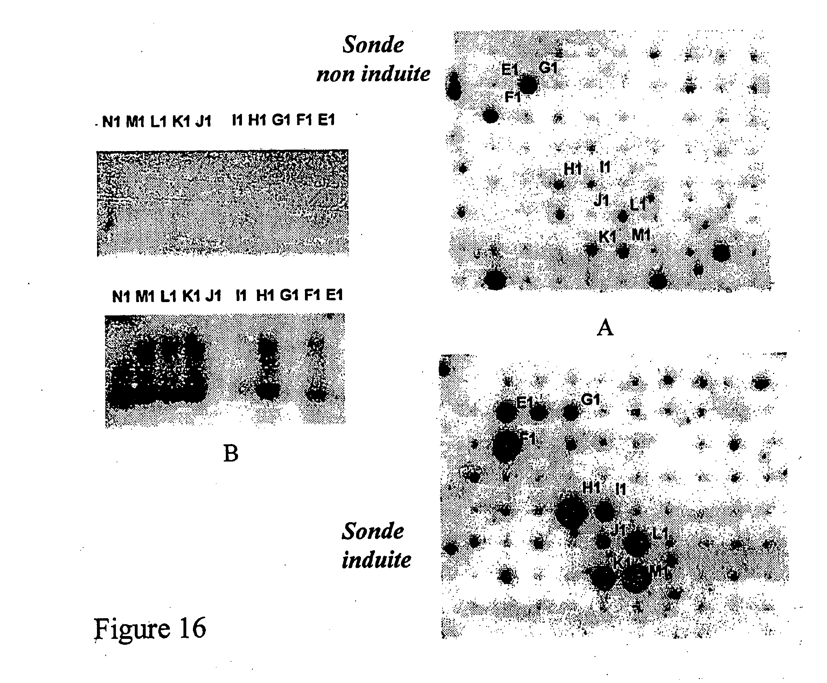

Un exemple de l'apport du criblage différentiel qualitatif à l'identification de gènes différentiellement épissés est fourni par l'application de DATAS à un modèle d'induction d'apoptose par induction de l'expression de la forme sauvage de p53. Ce modèle cellulaire a été établi par transfection d'un système d'expression inductible pour le gène suppresseur de tumeur p53. De façon à identifier les différences qualitatives qui sont associés spécifiquement à l'apoptose induite par p53, DATAS a été mise en oeuvre à partir des ARN messagers extraits de cellules induites et non induites. Pour ces expériences 200ng d'ARN polyA+ et 200ng d'ADNc ont été utilisés lors de la formation des hétéroduplex. Une centaine de clones a été obtenue à partir de chacune des hybridations croisées. L'hybridation de ces clones bactériens puis des fragments d'ADNc qu'ils contiennent avec des sondes représentatives des ARN messagers totaux des situations de départ a permis l'identification de séquences spécifiquement exprimées lors de la forte induction de p53 qui aboutit à la mort cellulaire (figure 16).

Ces fragments dérivent de séquences exoniques ou introniques qui modulent la qualité du message présent et permettent de proposer les domaines fonctionnels auxquels ils participent ou qu'ils interrompent comme des cibles d'intervention pour induire ou inhiber la mort cellulaire.

Une telle approche aboutit également à la constitution d'une paire de banques qui rassemblent des événements différentiellement épissés entre une situation non apoptotique et une situation apoptotique. Cette paire de banque peut être utilisée pour tester le pouvoir hybridant d'une sonde dérivée d'une autre situation physiopathologique ou d'un traitement particulier. Le résultat d'une telle hybridation donnera des indications sur l'engagement éventuel du programme d'expression génétique de la situation testée vers l'apoptose.

Comme il ressort de la description ci-avant, Ici est décrit également :
- toute sonde nucléique, tout oligonucléotide, tout anticorps dirigé contre une séquence identifiée par la technique décrite dans la présente demande et caractérisés en ce qu'ils permettent de caractériser une situation pathologique,
- l'utilisation des informations issues de l'utilisation des techniques décrites pour la recherche de molécules organiques à visée thérapeutique par la mise en place de criblages caractérisés en ce qu'ils ciblent des domaines splicés différentiellement entre une situation saine et une situation pathologique ou bien caractérisés en ce qu'ils sont basés sur l'inhibition des gains de fonctions acquis par la protéine résultant d'un épissage différentiel,
- l'utilisation des informations issues des techniques décrites dans la présente demande pour des applications de thérapie génique,
- l'utilisation d'ADNc transférés par thérapie génique caractérisés en ce qu'ils ont des propriétés antagonistes ou agonistes sur des voies de signalisation cellulaires définies,
- toute constitution et toute utilisation de banques moléculaires d'exons ou d'introns alternatifs à des fins:
   - de diagnostic ou de réactifs commerciaux pour la recherche
   - de créer ou de rechercher des molécules, polypeptides, acides nucléiques pour application thérapeutique.
- toute constitution et toute utilisation de banques virtuelles informatiques regroupant des exons ou introns alternatifs caractérisés en ce que ces banques permettent de concevoir des sondes nucléiques ou des amorces oligonucléotidiques dans le but de caractériser les épissages alternatifs qui différentient deux états physiopathologiques distincts.
   - toute composition pharmaceutique ou diagnostique comprenant des polypeptides, acides nucléiques sens ou anti-sens, ou des molécules chimiques capables d'interférer avec les produits d'épissage alternatifs mis en évidence et clonés par les techniques de l'invention,
   - toute composition pharmaceutique ou diagnostique comprenant des polypeptides, acides nucléiques sens ou anti-sens, ou des molécules chimiques capables de restaurer un épissage représentatifs d'une situation normale par opposition à l'événement alternatif caractéristique d'une situation pathologique.

### 5. DEREGULATIONS DES MÉCANISMES D'EPISSAGE DES ARNS PAR DES AGENTS TOXIQUES.

Cet exemple montre que les différences de formes et/ou profils d'épissages peut être utilisée comme marqueur pour le suivi et/ou la détection de toxicité et/ou d'efficacité de composés.

Les effets d'agents toxiques sur les dérégulations d'épissages des ARNs a été testé de la manière suivante. Des cellules hépatocytaires, HepG2, ont été traitées par différentes doses de trois composés toxiques (ethanol, camptothecine, PMA (Phorbol 12-Myristate 13-Acetate)). Deux tests de cytotoxicité (Bleu de Trypan, MTT) ont été réalisés à différents temps : 4h et 18h pour l'ethanol ; 4h et 18h pour la camptothecine ; 18h et 40h pour le PMA.

Le Bleu de Trypan est un colorant qui peut être incorporé par les cellules vivantes. Un simple comptage des cellules "bleues" et "blanches" sous microscope permet de déterminer le pourcentage de cellules vivantes après traitement ou pourcentage de survie. Les points sont effectués en triplicates.

Le test MTT est un test colorimétrique qui mesure la capacité des cellules vivantes à convertir les sels solubles de tetrazolium (MTT) en un précipité insoluble de formazan. Ces cristaux de formazan, bleu foncé, peuvent être dissous et leur concentration déterminée par mesure d'absorbance à 550 nm. Ainsi, après ensemencement de plaques 24 puits par 150000 cellules sur la nuit, puis traitement des cellules par les composés toxiques, est ajouté 50 µl de MTT (Sigma) (à 5 mg/ml dans du PBS). La réaction de formation des cristaux de formazan s'effectue en 5 h dans l'incubateur à CO2 (37°C, 5% CO2, 95% humidité). Après addition de 500 µl de solution de solubilisation (Hcl O, 1N dans Isopropanol-Triton X-100 (10%)), les cristaux sont dissous sous agitation et les absorbances mesurées à 550 à 660nm. Les points sont effectués en triplicates avec les contrôles (viabilité, mort cellulaire, blancs) appropriés.

Un test d'apoptose ou mort cellulaire programmée a également été réalisé par mesure de la fragmentation d'ADN via l'utilisation d'anticorps anti-histone et de mesures d'ELISA. Le test utilisé est Cell Death ELISA Plus de Roche.

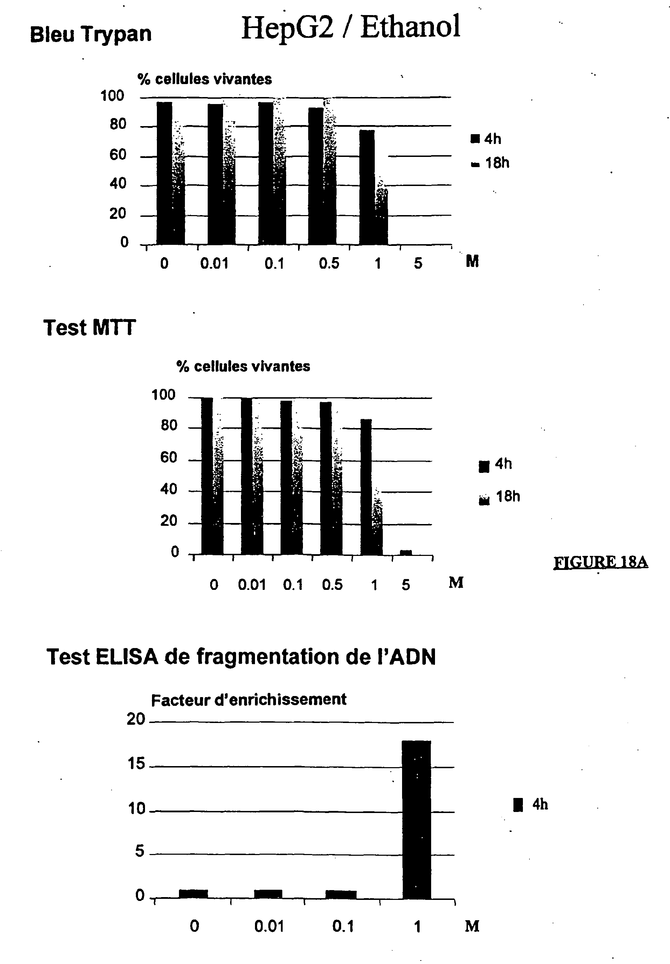

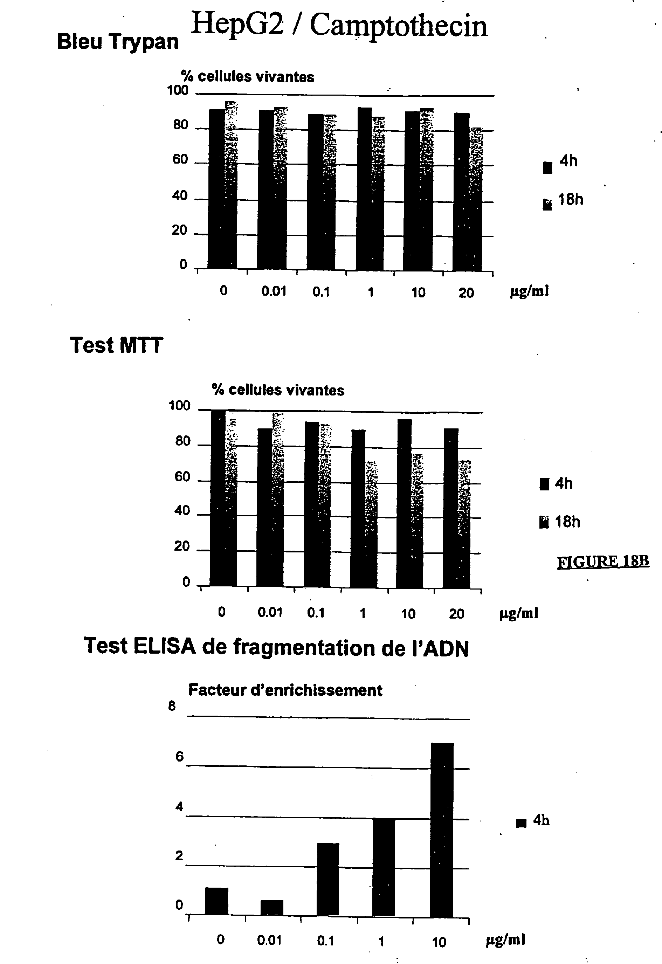

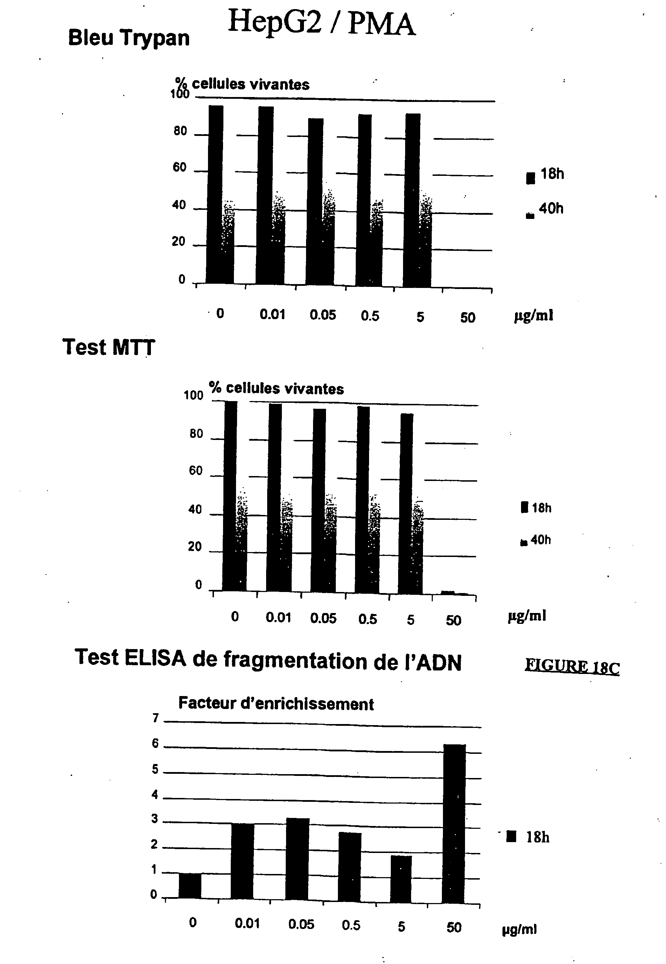

Les résultats de ces trois tests (Figures 18A,B,C) ont permis de déterminer que les doses suivantes :
- ethanol : 0,1 M
- camptothecine : 1 µg/ml
- PMA: 50 ng/ml
étaient bien inférieures aux IC50s mesurées.

Les cellules HepG2 ont ainsi été traitées par ces trois composés à ces trois doses pendant 4 h pour l'ethanol et la camptothecine et 18 h pour le PMA. Les ARNs messagers ont été purifiés par billes Dynal-Oligo-(dT) à partir d'ARNs totaux purifiés selon le kit Rneasy (Quiagen). Des synthèses d'ADNc ont été effectuées à partir de ces ARNs messagers et de la Transcriptase Inverse Superscript (Life Technologies) en utilisant des hexamères aléatoires comme amorces.

Ces premiers brins ont servi de matrices à des réactions d'amplification par PCR (94°C 1 mn, 55°C 1 mn, 72°C 1 mn, 30 cycles) à l'aide des amorces oligonucléotidiques suivantes :
MACH-α:
   5'-TGCCCAAATCAACAAGAGC-3' (SEQ ID NO: 11)
   5'-CCCCTGACAAGCCTGAATA-3' (SEQ ID NO: 12)

Ces amorces correspondent à des régions communes aux différentes isoformes décrites de MACH-α (1,2 et 3, amplifiant respectivement 595, 550 et 343 paires de bases). MACH-α (Caspase-8) est une protéase impliquée dans la mort cellulaire programmée (Boldin et.al, Cell (1996), 85, 803-815).
BCL-X:
   5' ATGTCTCAGAGCAACCGGGAGCTG 3' (SEQ ID NO: 13)
   5' GTGGCTCCATTCACCGCGGGGCTG 3' (SEQ ID NO: 14)

Ces amorces correspondent à des régions communes aux différentes isoformes décrites de bcl-X (bcl-XI, bcl-Xs, BCL-Xβ) (Boise et al, Cell (1993) 74, 597-608; U72398 (Genbank)) et doivent amplifier un fragment unique pour ces trois isoformes de 204 paires de bases.
FASR:
   5'-TGCCAAGAAGGGAAGGAGT-3' (SEQ ID NO: 15)
   5'-TGTCATGACTCCAGCAATAG-3' (SEQ ID NO: 16)

Ces amorces correspondent à des régions communes à certaines isoformes de FASR et doivent amplifier un fragment de 478 paires de bases pour la forme sauvage de FasR, 452 pour l'isoforme Δ8 et 415 pour l'isoforme ΔHM.

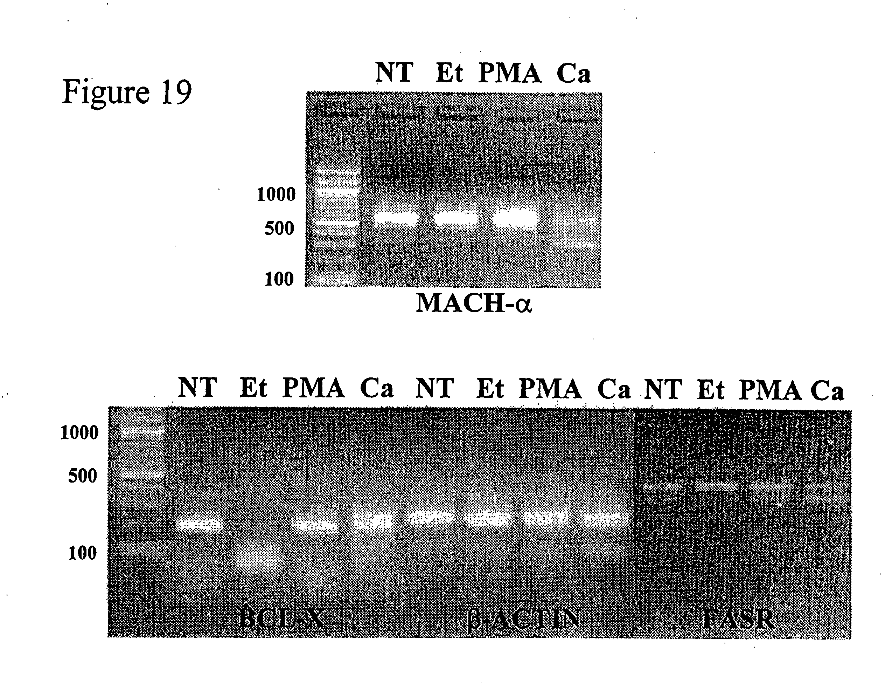

Les résultats rapportés sur la figure 19 indiquent que :
* la camptothécine induit une diminution de l'expression de l'isoforme MACH-α1 et une augmentation de l'isoforme MACH-α3.
* La camptothécine induit l'apparition d'une nouvelle isoforme de bcl-X (bande supérieure du doublet migrant vers 200 paires de bases).
* La camptothécine induit une diminution de la forme sauvage du récepteur de fas, remplacé par une expression d'une isoforme plus courte pouvant correspondre à Fas ΔHM.
* L'éthanol induit la disparition de bcl-x, remplacé par une isoforme plus courte.
* L'éthanol induit une augmentation de la forme longue, sauvage, du récepteur de fas, ceci aux dépens de l'isoforme plus courte.

Ces résultats démontrent que des traitements par des agents toxiques à des doses faibles peuvent induire des dysrégulations d'épissages alternatifs de certains ARNs, ceci de façon spécifique. L'identification de ces dysrégulations au niveau post-transcriptionnel, notamment par l'application de la technologie DATAS permet ainsi de définir un outil prédictif de la toxicité de molécules.

## Revendications

1. Composition comprenant un support et des oligonucléotides, **caractérisée en ce que** lesdits oligonucléotides sont simple brin, d'une longueur comprise entre 5 et 50 nucléotides, et sont spécifiques de variations dans les formes et/ou profils d'épissage de plusieurs gènes distincts, et **en ce que** lesdits oligonucléotides sont susceptibles d'être obtenus par un procédé comprenant :
(a) l'identification d'un événement d'épissage caractéristique d'une situation physiopathologique et de la séquence du domaine épissé ;
(b) la synthèse artificielle d'un ou plusieurs oligonucléotides correspondant à une ou plusieurs régions de ce domaine et permettant, par hybridation, de mettre en évidence la forme non épissée dans les ARN d'un échantillon test;
(c) la synthèse artificielle d'un ou plusieurs oligonucléotides correspondant à la région de jonction entre les deux domaines séparés par le domaine épissé, et permettant par hybridation de mettre en évidence la forme épissée dans les ARN d'un échantillon test;
(d) la reproduction des étapes (a) à (c) ci-dessus avec d'autres événements d'épissages caractéristiques de ladite situation physiopathologique ; et
(e) le transfert des oligonucléotides sur un support approprié.

2. Composition selon la revendication 1, **caractérisée en ce que** lesdits oligonucléotides sont d'une longueur de 25 nucléotides environ.

3. Composition selon la revendication 1 ou 2, **caractérisée en ce que** le support est composé d'un filtre, d'une membrane ou d'une puce.

## Patentansprüche

1. Zusammensetzung, umfassend einen Träger und Oligonukleotide, **dadurch gekennzeichnet, dass** die besagten Oligonukleotide einzelsträngig sind, eine Länge zwischen 5 und 50 Nukleotiden aufweisen und für Variationen in den Spleißformen und/oder -profilen mehrerer verschiedener Gene spezifisch sind, und dass die besagten Oligonukleotide mithilfe eines Verfahrens erhalten werden können, umfassend:
(a) die Identifizierung eines Spleißereignisses, das für einen physiopathologischen Zustand charakteristisch ist, und der Sequenz der gespleißten Domäne;
(b) die künstliche Synthese eines oder mehrerer Oligonukleotide, die einer oder mehreren Regionen dieser Domäne entsprechen und die mittels Hybridisierung den Nachweis der ungespleißten Form in den RNAs einer Testprobe erlauben;
(c) die künstliche Synthese eines oder mehrerer Oligonukleotide, die der Verknüpfungsregion zwischen den zwei Domänen entsprechen, die durch die gespleißte Domäne getrennt sind, und die mittels Hybridisierung den Nachweis der gespleißten Form in den RNAs einer Testprobe erlauben;
(d) die Reproduktion der Schritte (a) bis (c) oben mit anderen Spleißereignissen, die für den besagten physiopathologischen Zustand charakteristisch sind; und
(e) die Übertragung der Oligonukleotide auf einen geeigneten Träger.

2. Zusammensetzung gemäß Anspruch 1, **dadurch gekennzeichnet, dass** die besagten Oligonukleotide eine Länge von ungefähr 25 Nukleotiden aufweisen.

3. Zusammensetzung gemäß Anspruch 1 oder 2, **dadurch gekennzeichnet, dass** der Träger aus einem Filter, einer Membran oder einem Chip besteht.

## Claims

1. A composition comprising a support and oligonucleotides, wherein said oligonucleotides are single-stranded, have a length comprised between 5 and 50 nucleotides, and are specific of variations in the splicing forms and/or patterns of several distinct genes, and wherein said oligonucleotides are obtainable by a process comprising:
(a) identifying a splicing event characteristic of a physiopathological situation and the sequence of the spliced domain;
(b) synthesizing artificially one or several oligonucleotides corresponding to one or several regions of this domain and allowing the identification of the unspliced form in the RNAs of a test sample through hybridization ;
(c) synthesizing artificially one or several oligonucleotides corresponding to the junction region between two domains separated by the spliced domain and allowing the identification of the spliced form in the RNAs of a test sample through hybridization;
(d) repeating steps (a) to (c) above with other splicing events characteristic of said physiopathological situation; and
(e) transferring the oligonucleotides on a suitable support.

2. Composition according to claim 1, wherein said oligonucleotides have a length of about 25 nucleotides.

3. Composition according to claim 1 or 2, wherein the support is composed of a filter, a membrane or a chip.
